(19) Europäisches Patentamt
European Patent Office
Office européen des brevets

(11) **EP 2 535 041 A1**

(12) **EUROPEAN PATENT APPLICATION**

(43) Date of publication:
**19.12.2012 Bulletin 2012/51**

(51) Int Cl.:
*A61K 9/00* (2006.01)   *A61L 27/14* (2006.01)
*C08F 283/00* (2006.01)   *C08J 3/24* (2006.01)
*C08L 5/00* (2006.01)   *C08L 89/00* (2006.01)

(21) Application number: **12005906.8**

(22) Date of filing: **11.09.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.09.2005 US 715411 P**

(62) Document number(s) of the earlier application(s) in accordance with Art. 76 EPC:
**06790690.9 / 1 934 289**

(71) Applicants:
• **Ottawa Health Research Institute**
  **Ottawa, Ontario K1Y 4K9 (CA)**
• **University Of Ottawa**
  **Ottawa, Ontario K1N 6N5 (CA)**

(72) Inventors:
• **May, Griffith**
  **Carp**
  **ON K1Y 1L0 (CA)**

• **Fengfu, Li**
  **Glouchester**
  **ON K1J 1B4 (CA)**
• **Wenguang, Liu**
  **Tianjin (CN)**
• **Mehrdad, Rafat**
  **Gatineau,**
  **QC J9H 7K7 (CA)**

(74) Representative: **Grünecker, Kinkeldey,**
  **Stockmair & Schwanhäusser**
  **Leopoldstrasse 4**
  **80802 München (DE)**

Remarks:
This application was filed on 16-08-2012 as a divisional application to the application mentioned under INID code 62.

(54) **Interpenetrating networks, and related methods and composition**

(57) The present invention provides interpenetrating polymeric networks (IPNs), and related methods and compositions. The hydrogel material of this invention comprises an interpenetrating network of two or more polymer networks, wherein at least one of the polymer networks is based on a biopolymer. Also provided is a method of producing the hydrogel material comprising, combining a first polymeric network with a second polymeric network, wherein the first polymeric network or the second polymeric network is based on a biopolymer. The present application also discloses devices manufactured from the IPN hydrogel material and uses thereof.

**FIGURE 2**

**Description**

## FIELD OF THE INVENTION

The present invention relates to a hydrogel material comprising an interpenetrating polymeric network. More particularly, the present invention relates to hydrogel material comprising an interpenetrating polymeric network in which at least component network is based on a bioolymer and uses thereof, as well as devices manufactured from the hydrogel material.

## BACKGROUND

Tissue engineering is a rapidly growing field encompassing a number of technologies aimed at replacing or restoring tissue and organ function. The key objective in tissue engineering is the regeneration of a defective tissue through the use of materials that can integrate into the existing tissue so as to restore normal tissue function. Tissue engineering, therefore, demands materials that can support cell over-growth, in-growth or encapsulation and, in many cases, nerve regeneration.

United States Patent No. 5,716,633 describes a collagen-hydrogel promoting epithelial cell growth, made from collagen (~0.12-0.14%(w/w)) and 2-hydroxylethyl methacrylate (HEMA), using ammonium persulfate and sodium metabisulfate as a free radical initiator at 38°C in contact lens molds. Ethylene glycol dimethacrylate was used as a cross-linking agent to cross link HEMA only. In this patent, the collagen concentration is very low, and the collagen is not cross-linked. In such a system, collagen can leach out in to the surrounding aqueous media.

United States Patent No. 4,388,428 describes biologically stabilized hydrogels as contact lens material, composed of collagen and ethylenically unsaturated compounds and cross-linking agents, e.g., N-isopropylacrylamide and N,N-methylenebisacrylamide via $^{60}$Co irradiation. There is some bonding between collagen and the synthetic polymer. The final collagen content is about 7% w/w. In this gel system only the ethylenically unsaturated compound is effectively cross-linked; the collagen is only slightly cross-linked by gamma irradiation of 1.0Mrd total dose.

United States Patent No. 4,452,929 describes an aqueous coating composition with a collagen concentration of about 1.5% in the final collagen-ethylenically unsaturated compound hydrogel.

Examples of vision enhancing ophthalmic materials that are non-biodegradable and allow regeneration of corneal cells and nerves when implanted have been reported. However, despite these properties, these materials still lack the elasticity and optimum toughness for easy handling during surgery, especially under sub-optimal conditions such as in developing countries.

Accordingly, there remains a need for materials that can be used in ophthalmic devices and that have the required elasticity and toughness for handling during surgery.

This background information is provided for the purpose of making known information believed by the applicant to be of possible relevance to the present invention. No admission is necessarily intended, nor should be construed, that any of the preceding information constitutes prior art against the present invention.

## SUMMARY OF THE INVENTION

An object of the present invention is to provide interpenetrating polymeric networks (IPNs), and related methods and compositions.

In accordance with one aspect of the present invention, there is provided a hydrogel material comprising an interpenetrating network of two or more polymer networks, wherein at least one of the polymer networks is based on a biopolymer.

In accordance with another aspect of the present invention, there is provided a method of producing a hydrogel material according to the present invention, the method comprising, combining a first polymeric network with a second polymeric network, wherein the first polymeric network or the second polymeric network is based on a biopolymer.

In accordance with another aspect of the invention, there is provided a kit for producing a hydrogel material according to the present invention, the kit comprising, (i) an interpenetrating polymeric networks of two or more polymeric networks, wherein at least one of the polymeric networks is based on a biopolymer; and (ii) instructions for the production thereof.

In accordance with another aspect of the present invention, there is provided devices manufactured from the IPN hydrogel material, including, but not limited to implants (e.g., corneal implants), corneal onlays, nerve conduit, blood vessels, drug delivery device and catheters, therapeutic lens, intraocular lens, and methods of manufacture thereof.

## EMBODIMENTS OF THE INVENTION

The present invention provides in embodiments:

1. A hydrogel material comprising an interpenetrating network of two or more polymer networks, wherein at least one of the polymer networks is based on a biopolymer.

2. The hydrogel material according to embodiment 1, wherein the biopolymer is, denatured gelatin, fibrin-fibrinogen, elastin, glycoprotein, polysaccharide, glycosaminoglycan, proteoglycan, or oxidized polysaccharide or any combination thereof.

3. The hydrogel material according to embodiment 2, wherein the collagen is Type I collagen, Type II collagen, Type III collagen, Type IV collagen, Type collagen V, Type VI collagen, denatured collagen or recombinant collagen.

4. The hydrogel material according to embodiment 2, wherein the polysaccharide is alginate, chitosan, N-carboxymethyl chitosan, O-carboxymethyl chitosan, N,O-carboxymethyl chitosan, hyaluronic acid or chondroitin sulphates.

5. The hydrogel material according to embodiment 2, wherein the oxidized polysaccharide is oxidized chondroitin sulphate, oxidized alginate or oxidized hyaluronic acid.

6. The hydrogel material according to embodiment 1, wherein at least one of the polymer networks is based on a synthetic polymer.

7. The hydrogel according to embodiment 6, wherein the synthetic polymer is alkyl acrylamide, water soluble polyethylene glycol diacrylate, acrylic acid and its derivatives, alkyl acylate, methylacrylic acid and its derrivatives, alkyl methacrylate, 2-hydroxyethyl methacrylate, 2-methacryloyloxyethyl phosphorylcholine, vinyl pyrrolidone or glycomonomer.

8. The hydrogel material according to any one of embodiments 1 to 7, wherein the material is characterized by at least one of low cytotoxicity, no cytotoxicity, an ability to facilitate cell and/or nerve growth, moldability, an ability to withstand handling, implantation, suturing and/or post-installation wear and tear.

9. The hydrogel material according to anyone of embodiments 1 to 8 additionally comprising a bioactive agent or a drug.

10. The hydrogel material according to anyone of embodiments 1 to 8, for use as an ophthalmic onlay or implant.

11. The hydrogel material according to anyone of embodiments 1 - 8 for use in drug delivery.

12. A method of producing a hydrogel material according to anyone of embodiments 1 - 10, the method comprising, combining a first polymer network with a second polymer network, wherein at least one of the first polymer network and the second polymer network is based on a biopolymer and maintains the resultant reaction mixture under conditions suitable for formation of an interpenetrating network.

13. The method according to embodiment 10, wherein said first and second polymer networks are combined with at least one cross-linking agent.

14. The method according to embodiment 12 or 13, wherein the reaction mixture is maintained at an acidic pH.

15. The method according to anyone of embodiments 12 to 14, wherein the reaction mixture is placed in a mold an allowed to cure.

16. A device comprising the hydrogel material according to anyone of embodiments 1 - 11, which device is suitable for administration to a mammal.

17. The device according to embodiments 16, which is an ophthalmic device.

18. The device according to embodiment 16 or 17, wherein said device comprises a bioactive agent or a drug for delivery to said mammal.

19. The device according to embodiment 18, wherein said bioactive agent or drug is dispersed within the hydrogel.

20. The device according to embodiment 18, wherein the drug is contained within nano- or microspheres dispersed within the hydrogel material.

21. The device according to embodiment 19, wherein the bioactive agent is a growth factor, retinoid, enzyme, cell adhesion factor, extracellular matrix glycoprotein, hormone, osteogenic factor, cytokine, antibody, antigen, biologically active protein, pharmaceutical compound, peptide, fragments or motifs derived from biologically active protein, anti-bacterial agent or anti- viral agents.

## BRIEF DESCRIPTION OF THE FIGURES

Figure 1 is a photograph of a NiColl20/MPC IPN hydrogel according to one embodiment of the present invention.

Figure 2 graphically depicts the mechanical properties (strength, elongation, and toughness) of two IPNs according to specific embodiments of the present invention;

Figure 3 graphically depicts the optical properties (% light transmission) for IPN-I and IPN-II compared to those for Control-I, Control-II, human and rabbit corneas.

Figure 4 graphically depicts growth of Human Epithelial Cells on (a) IPN-I and (b) IPN-II hydrogels.

Figure 5 depicts nerve growth on the surface of IPN-II (EP10-11) hydrogel.

Figure 6 is a photograph of a collagen-synthetic copolymeric IPN.

Figure 7 is a photograph of the above polymer (EP10-11) implanted into the cornea of a Yucatan mini-pig by lamellar keratoplasty (partial thickness graft).

Figure 8 depicts a chondroitin sulphate-based material to deliver endothelial progenitor cells (EPCs) into a muscle test system and obtaining incorporation of labelled EPCs (labelled green) into blood vessels via angiogenesis, (A) Injection site (arrow) of EPCs (green labeled) in skeletal muscle from rat ischemic hindlimb, (B) Magnified image of EPCs (arrows) migrating from injected matrix into the tissue, (C) EPCs were observed within blood vessel structures (arrows).

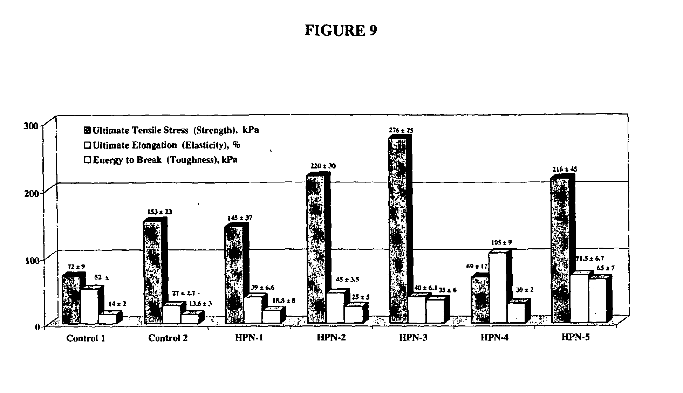

Figure 9 graphically depicts the mechanical properties (strength, elongation, and toughness) of HPN-3 to HPN-5 materials compared to Control-1, Control-2, HPN-1 (IPN-I), and HPN-2 (IPN-II);

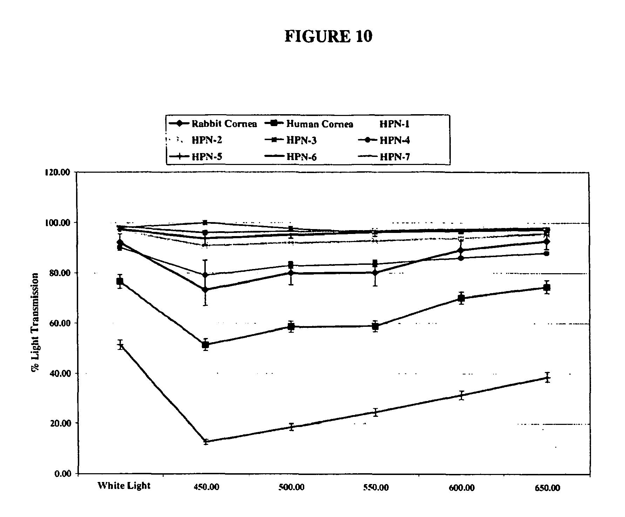

Figure 10 graphically depicts the optical properties (% light transmission) for HPN-3 to HPN-7 compared to those for HPN-1 (IPN-I), HPN-2 (IPN-II), and human and rabbit corneas.

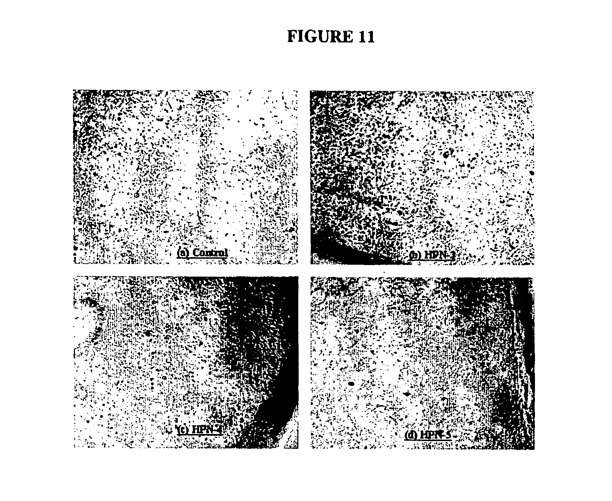

Figure 11 depicts growth of Human Epithelial Cells on (a) Culture dish control surface, (a) HPN-3, (b) HPN-4, and (c) HPN-5 hydrogels on day 6 post seeding;

Figure 12 graphically depicts the maximum strength hydrogels with different Collagen to DMA ratios in kPa ($\pm$ standard deviation);

Figure 13 graphically depicts the percent breaking strain of hydrogels for each Collagen to DMA ratio ($\pm$ standard deviation);

Figure 14 graphically depicts the modulus for Collagen to DMA ratios in kPa ($\pm$ standard deviation);

Figure 15 graphically depicts white light transmission of collagen-DMA hydrogels;

Figure 16 shows epithelium cell *in vitro* growth on collagen-DMA (3:1 w/w) hydrogels, optical images at day 3 showing cell confluence; and

Figure 17 is a Scanning Electron Micrograph image of microspheres of alginate (average diameter: 300 micron).

Figure 18 graphically depicts *in vitro* biodegradation of collagen and IPN hydrogels.

Figure 19 shows a comparison of the IPN (right hand column) with crosslinked recombinant human collagen (centre column). The left hand column shows untreated control.

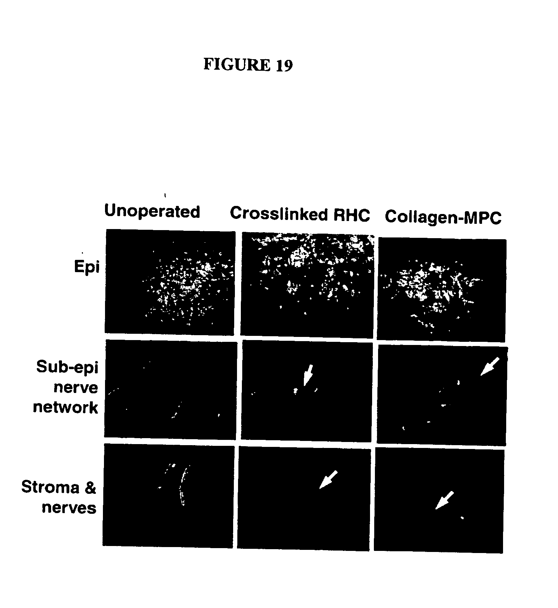

Figure 19 shows *In vivo* confocal images of typical implants at six month post-operative compared with a set of typical untreated, contralateral, control corneas. Both (EDC/NHS) crosslinked recombinant human collagen and medical grade porcine collagen-MPC IPNs show that the re-growth of nerves into the stroma and sub-epithelial nerve network (arrows).

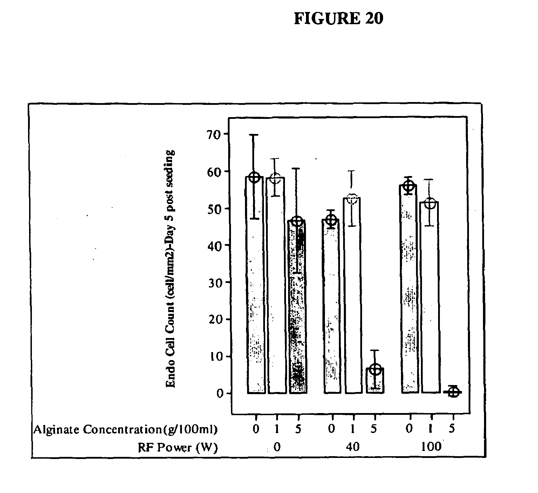

Figure 20 shows the effect of alginate-grafting onto plasma treated collagen/chitosan IPN on endothelium adhesion proliferation. Group A: Radio Frequency power (RFP) =0 w; Group B: RFP=40 w; Group C: RFP=100w. Blue bars: 0% alginate; Green bars: 1% alginate; Orange bars: 5%.

## DETAILED DESCRIPTION OF THE INVENTION

DEFINITIONS Unless defined otherwise, all technical and scientific terms used herein have the same meaning as commonly understood by one of ordinary skill in the art to which this invention pertains.

The term "hydrogel," as used herein, refers to a cross-linked polymeric material which exhibits the ability to swell in water or aqueous solution without dissolution and to retain a significant portion of water or aqueous solution within its structure.

The term "polymer," as used herein, refers to a molecule consisting of individual monomers joined together. In the context of the present invention, a polymer may comprise monomers that are joined "end-to-end" to form a linear molecule, or may comprise monomers that are joined together to form a branched structure.

The term "bio-polymer," as used herein, refers to a naturally occurring polymer. Naturally occurring polymers include, but are not limited to, proteins and carbohydrates. The term "bio-polymer" also includes derivatised forms of the naturally occurring polymers that have been modified to facilitate cross-linking to a synthetic polymer of the invention.

The term "synthetic polymer," as used herein, refers to a polymer that is not naturally occurring and that is produced by chemical or recombinant synthesis.

The term "interpenetrating network" or "IPN", as used herein, refers to an interpenetrating polymeric network, which is a combination of two or more polymers in which each polymer forms a network. There is entanglement and interactions between the networks. When swollen in a solvent, none of the polymers will dissolve in the solvent.

As used herein, "optically clear" refers to at least 85% transmission of white light. In certain embodiments, "optically clear" refers to optical clarity that is equivalent to that of a healthy cornea, for example, having greater than 90% transmission of white light and less than 3% scatter.

As used herein, a "comeal onlay" is an ophthalmic implant or device configured, in size and shape, to be located between the epithelium or an epithelial cell layer and the Bowman's membrane in an eye, of a human or animal. In comparison, a "contact lens" is configured to be located over the epithelium of an eye. A corneal onlay may rest entirely over the Bowman's membrane, or it may include one or more portions that extend into Bowman's membrane. Such portions constitute a minor portion of the device, such as less than 50% of the area or volume of the device.

As used herein, a "corneal inlay" is a device or implant configured to be placed in the stroma of an eye. Corneal inlays may be placed in the stroma by forming a flap or a pocket in the stroma. Corneal inlays are placed below the Bowman's membrane of an eye.

As used herein, a "full-thickness corneal implant" refers to a device that is configured to replace all or part of an unhealthy cornea of an eye located anterior to the aqueous humour of the eye.

## IPN Hydrogel Material

The IPN hydrogel material of the present invention comprises an IPN that is suitable for use in a variety of applications, including, but not limited to, clinical, therapeutic, prophylactic or cosmetic applications. The IPN hydrogel material can be used to replace, restore and/or augment tissue and/or organ function in a subject in need thereof.

The IPN hydrogel material of the present invention is characterized by low cytotoxicity or no cytotoxicity, ability to facilitate cell and/or nerve growth, and/or moldability. The material also has sufficient mechanical and structural properties to permit handling, implantation, and the like, which may include suturing, and post-installation wear and tear. In accordance with one embodiment of the present invention, devices made from the IPN hydrogel material are produced using molds. Such devices include, but are not limited to, molded ophthalmic onlays and implants, which are formed to the desired size and shape.

In accordance with a specific, non-limiting example of the present invention, the IPN material is used in ophthalmic devices, wherein the material can provide one or more of the following benefits to an individual to whom the device is fitted: (i) a desired refractive index, (ii) a desired optical clarity (for visible light, optical transmission and light scattering equal to or better than those of healthy human cornea material of comparable thickness), (iii) a desired optical power, such as a vision enhancing optical power, (iv) enhanced comfort, (v) enhanced corneal and epithelial health, and (vi) therapeutic benefit, for example, in the treatment of a disease, disorder or traumatic injury of an eye. In accordance with this embodiment, the material of the present invention can be made transparent, or optically clear. The material can also be molded to include a vision corrective curvature.

The material of the present invention is suitable for use in ophthalmic devices, in part, because it is (i) shapeable, such as moldable, to form a matrix with an acceptable optical power, (ii) effective in facilitating nerve growth through and/or over the device, and (iii) can be made optically clear or visually transparent. When the device is a corneal onlay, the device is effective in facilitating re-epithelialization over the anterior surface of the device.

The IPN material of the present invention can be manufactured to permit gas or nutrient diffusion as required for its particular application. For example, in the case of corneal onlays, the material from which the onlay is produced provides for or permits gas and nutrient exchange between the Bowman's membrane and epithelium to maintain a viable, fully functioning epithelium. Such nutrients include glucose and factors or agents to promote or enhance the survival, growth, and differentiation of cells, such as epithelial cells. The exchange should be comparable to or better than that of a healthy human cornea. The permeability of the material to nutrients and/or drugs can be monitored using conventional techniques. In addition, the movement of the nutrients and/or drugs through the material should not cause the optical properties of the material to change significantly. The onlays or lenticules are fully biocompatible, allow rapid epithelial adhesion to the onlay, and permit restoration of nerve innervation and sensitivity, for example touch sensitivity.

The IPN hydrogel material of the present invention comprises a combination of two or more polymeric networks. At least one of the polymeric networks is formed from a bio-polymer. The second polymer network is formed from either a synthetic polymer or a second bio-polymer. The material can comprise a third, or more, polymeric network formed by sequential IPN. For example, an IPN material can swell in a third monomer with cross-linker to form an additional network after curing. The third monomer can be the same as the first or the second monomer.

Bio-polymers

Bio-polymers are naturally-occurring polymers and their derivatives, such as proteins and carbohydrates. In accordance with the present invention, the material comprises a bio-polymer or a derivatised version thereof, in the form of a network. Examples of suitable bio-polymers for use in the present invention include, but are not limited to, collagens (including Types I, II, III, IV, V and VI), denatured collagens (or gelatins), recombinant collagens, fibrin-fibrinogen, elastin, glycoproteins, polysaccharides such as, but not limited to, alginate, chitosan, N-carboxymethyl chitosan, O-carboxymethyl chitosan, N,O-carboxymethyl chitosan, hyaluronic acid, chondroitin sulphates and glycosaminoglycans (or proteoglycans), oxidized polysaccharides such as, but not limited to oxidized chondroitin sulphate, oxidized alginate and oxidized hyaluronic acid.

Suitable bio-polymers for use in the invention can be purchased from various commercial sources or can be prepared from natural sources by standard techniques.

A bio-polymer or derivative thereof is selected based on one or more of the following properties: (1) the bio-polymer is bio-compatible and optionally promotes cell adhesion and growth and/or promotes nerve growth; (2) the bio-polymer includes reactive groups which can be cross-linked by a variety of cross-linking agents, for example, but not limited to, EDC/NHS chemistry to form one component of an IPN; (3) the bio-polymer can be cross-linked to form a hydrogel, i.e. one component of a network via chelating ions or physically cross-linked by pH or temperature. In one example, alginate is cross-linked forming a hydrogel by adding $Ca^{2+}$ into alginate aqueous solution; (4) a derivitised bio-polymer, for example oxidized polysaccharides (oxidized chondroitin sulfate bears aldehyde groups), can be chosen to crosslink another bio-polymer, such as collagen, to form one component of the IPN; (5) the bio-polymer may form a transparent IPN with the synthetic polymer for ophthalmic device use. However, non-transparent IPN may also be used in other applications, such as a sclera patch or in other tissue engineering areas.

Synthetic polymers

Monomers that form synthetic polymers include, for example, but not limited to various alkyl acrylamide, water soluble polyethylene glycol diacrylate, acrylic acid and its derivatives, alkyl acylate, methylacrylic acid and its derrivatives, alkyl methacrylate, 2-hydroxyethyl methacrylate, 2-methacryloyloxyethyl phosphorylcholine, vinyl pyrrolidone, glycomonomer (herein refers to a polymerizable monomer which is derivatised monosaccharide or a derivitised oligosaccharide, for example, glycosyloxyethyl methacrylate and 2-methacryloxyethyl glucoside). The resultant polymers should be biocompatible, biosafe and miscible with bio-polymers.

The starting monomers are hydrophilic and usually contain polymerizable double bonds, the polymerization should occur at a temperature below about 37°C, or below the denaturation temperature of the protein, in some cases such as when the a protein as a biopolymer such as collagen is used as the other component to form the IPN.

Bio-active Agents

The IPN hydrogel material of the present invention may be manufactured to include one or more bio-active agents. Selection of the appropriate bio-active agent or combination of agents is based on the application of the material. Non-limiting examples of bioactive agents that may be incorporated into the material include, for example, growth factors, retinoids, enzymes, cell adhesion factors, extracellular matrix glycoproteins (such as laminin, fibronectin, tenascin and the like), hormones, osteogenic factors, cytokines, antibodies, antigens, and other biologically active proteins, certain pharmaceutical compounds, as well as peptides, fragments or motifs derived from biologically active proteins. Bioactive agents also include anti-bacterial and anti-viral agents.

**Method of Preparing the IPN Based Hydrogel Material**

As discussed above, IPN is an interpenetrating polymeric network. The reactants are added in one pot in a sequence, and the crosslinking reaction occurs simultaneously. For example to make NiColl/MPC IPN (example I): Collagen is cross-linked by EDC/NHS forming one polymeric network; MPC is cross-linked by PEG-diacrylate forming another polymeric network. These two polymeric networks are interpenetrating each other forming an IPN. To make a thermodynamic compatible interpenetrating polymer network, which is important to the mechanical and, optionally, optical properties of the devices, the relative amounts of monomer and polymer as well as crosslinking agents are controlled in a certain range. An excessive amount of one component may result in large size of a micro domain, causing an evident phase separation, which will deteriorate the properties of devices. Thus selecting the amount of components in IPN should first meet the basic requirements of IPN. The ratio of the components can be adjusted in order to make a transparent IPN for ocular application. The ratio of components can also be adjudted to meet mechanical properties of the final hydrogel.

**Testing the IPN Based Hydrogel Material**

In accordance with the present invention, in order to be suitable for *in vivo* implantation for tissue engineering purposes, the hydrogel material, with or without added bioactive agents, must maintain its form at physiological temperatures, be adequately robust for handling and suturing, be substantially insoluble in water, support the growth of cells and be inert for using as blood vessel, catheter or intraocular lens for cataract surgery. It may also be desirable for the material to support the growth of nerves. It will be readily appreciated that for certain specialised applications, the material may require other characteristics. For example, for surgical purposes, the material may need to be relatively flexible as well as strong enough to support surgical manipulation with suture thread and needle, and for ophthalmic applications, such as cornea repair or replacement, the optical clarity of the material will be important. The components of the IPN material and their relative amounts are selected to provide the required characteristics.

Physical / Chemical testing

When used for tissue engineering applications, the material must exhibit the mechanical properties necessary to prevent tearing or rupturing when subjected to surgical procedures and to provide adequate support for cell growth in and/or around the material once in place. The ability of material to resist shearing forces and tearing is related to its intrinsic mechanical strength, the form and thickness of the material and the tension being applied.

The ability of the material to withstand shearing forces, or tearing can be roughly determined by applying forces in opposite directions to the specimen using two pairs of forceps. Alternatively, a suitable apparatus can be used to quantitatively measure the ability of the material to withstand shearing forces. Tensiometers for this purpose are available commercially, for example, from MTS, Instron, and Cole Parmer.

For testing, the material can be formed into sheets and then cut into appropriately sized strips. Alternatively, the material can be molded into the desired shape for tissue engineering purposes and the entire molded material can be tested. To calculate tensile strength, the force at rupture, or "failure," of the material is divided by the cross-sectional area of the test sample, resulting in a value that can be expressed in force (N) per unit area. The stiffness (modulus) of the material is calculated from the slope of the linear portion of the stress/strain curve. Strain is the real-time change in length during the test divided by the initial length of the test sample before the test begins. The strength at rupture is the final length of the test sample when it ruptures minus the length of the initial test sample, divided by this initial length.

One skilled in the art will appreciate that because of the softness of hydrogels and exudation of the aqueous component when clamped, meaningful tensile data can be difficult to obtain from hydrogels. Quantitative characterisation of tensile strength in hydrogels can be achieved, for example, through the use of suture pull-out measurements on molded material samples. Typically, a suture is placed about 2 mm from the edge of a test sample and the peak force that needs to be applied in order to rip the suture through the sample is measured. For example, for a test sample of material intended for ophthalmic applications that has been molded in the shape and thickness of a human cornea, two diametrically opposed sutures can be inserted into the material, as would be required for the first step in ocular implantation. The two sutures can then be pulled apart at about 10 mm/min on a suitable instrument, such as an Instron Tensile Tester. Strength at rupture of the material is calculated, together with elongation at break and elastic modulus [see, for example, Zeng et al., J. Biomech., 34:533-537 (2001)]. It will be appreciated by those skilled in the art that, for that material intended for surgical applications, the material need not be as strong (i.e., have the same ability to resist tearing) as mammalian tissue. The determining factor for the strength of the material in such applications is whether or not it can be sutured in place by a careful and experienced surgeon.

If desired, the lower critical solution temperature (LCST) of the hydrogel material can be measured using standard techniques. For example, LCST can be calculated by heating samples of the matrix at about 0.2°C per minute and visually observing the cloud point (see, for example, H. Uludag, et al., J. Appl. Polym. Sci. 75:583 - 592 (2000)).

Permeability of the material can be determined by assessing the glucose permeability coefficient and/or the average pore sizes for the material using standard techniques such as PBS permeability assessment using a permeability cell and/or atomic force microscopy.

Optical transmission and light scatter can also be measured for material intended for ophthalmic applications using a custom-built instrument that measures both transmission and scatter (see, for example, Priest and Munger, Invest. Ophthalmol. Vis. Sci. 39: S352 (1998)

*In vitro* Testing

It will be readily appreciated that the material must be non-cytotoxic or minimally/acceptably cytotoxic and biocompatible in order to be suitable for *in vivo* use. The cytotoxicity of the material can be assessed using standard techniques such as the Ames assay to screen for mutagenic activity, the mouse lymphoma assay to screen for the ability of the material to induce gene mutation in a mammalian cell line, *in vitro* chromosomal aberration assays using, for example, Chinese hamster ovary cells (CHO) to screen for any DNA rearrangements or damage induced by the matrix. Other assays include the sister chromatid assay, which determines any exchange between the arms of a chromosome induced by the matrix and *in vitro* mouse micronucleus assays to determine any damage to chromosomes or to the mitotic spindle. Protocols for these and other standard assays are known in the art, for example, see *OECD Guidelines for the Testing of Chemicals* and protocols developed by the ISO.

The ability of the material to support cell growth can also be assessed *in vitro* using standard techniques. For example, cells from an appropriate cell line, such as human epithelial cells, can be seeded either directly onto the material or onto an appropriate support surrounding the material. After growth in the presence of a suitable culture medium for an appropriate length of time, confocal microscopy and histological examination of the material can be conducted to determine whether the cells have grown over the surface of and/or into the material.

The ability of the material to support in-growth of nerve cells can also be assessed in *vitro*. For example, a nerve source, such as dorsal root ganglia, can be embedded into an appropriate support surrounding the material or directly inserted into the material. An example of a suitable support would be a soft collagen based gel. Cells from an appropriate cell line can then be seeded either directly onto the material or onto an appropriate support surrounding the material and the material can be incubated in the presence of a suitable culture medium for a pre-determined length of time. Examination of the material, directly and/or in the presence of a nerve-specific marker, for example by immunofluorescence using a nerve-specific fluorescent marker and confocal microscopy, for nerve growth will indicate the ability of the material to support neural in-growth.

Growth supplements can be added to the culture medium, to the material or to both in experiments to assess the ability of the material to support cell growth. The particular growth supplements employed will be dependent in the type of cells being assessed (e.g., in view of the intended application of the hydrogel material) and can be readily determined by one skilled in the art. Suitable supplements for nerve cells, for example, include laminin, retinyl acetate, retinoic acid and nerve growth factors for nerve cells.

*In vivo* Testing

In order to assess the biocompatibility of the material and its ability to support cell growth in vivo, the material can be implanted into an appropriate animal model for immunogenicity, inflammation, release and degradation studies, as well as determination of cell growth. Suitable control animals may be included in the assessment. Examples of suitable controls include, for example, unoperated animals, animals that have received allografts of similar dimensions from a donor animal and/or animals that have received implants of similar dimensions of a standard, accepted implant material.

At various stages post-implantation, biopsies can be taken to assess cell growth over the surface of and/or into the implant and histological examination and immunohistochemistry techniques can be used to determine whether nerve in-growth has occurred and whether inflammatory or immune cells are present at the site of the implant. For example, various cell-specific stains known in the art can be used to assess the types of cells present as well as various cell-specific antibodies, such a anti-neurofilament antibodies that can be used to indicate the presence or absence of nerve cells. In addition, measurement of the nerve action potentials using standard techniques will provide an indication of whether the nerves are functional. *In vivo* confocal microscopic examination can be used to monitor cell and nerve growth in the animal at selected post-operative times. Where appropriate, touch sensitivity can be measured by techniques known in the art, for example, using an esthesiometer. Restoration of touch sensitivity indicates the regeneration of functional nerves.

Applications

The present invention provides an IPN hydrogel material that is biocompatible and non-cytotoxic (or minimally cytotoxic) and, therefore, suitable for use as a scaffold to allow tissue regeneration *in vivo.* For example, the material can be used for implantation into a patient to replace tissue that has been damaged or removed, for wound coverage, as a tissue sealant or adhesive, as a skin substitute or cornea substitute, or as a corneal veneer. The material can be molded into an appropriate shape prior to implantation, for example it can be pre-formed to fill the space left by damaged or removed tissue. The material can also be used as a Support/scaffold that does not necessarily allow tissue regeneration, which may be desirable for example when used as intraocular lens or therapeutic lens.

In addition, the IPN hydrogel material of the present invention can be used in, for example, i) transplantation using corneal implants; ii) refractive correction by way of corneal inlays, onlays, implantable contact lenses, IOLs; iii) cataract surgery - IOLs; iv) reconstruction of skin; optionally in combination with fibrin (to induce vascular ingrowth via angiogenesis); v) delivery of drugs, peptides or growth factors for stimulation of stem cell growth, and differentiation (particular examples of this use of the material of the present invention include, as bandage contact lenses or implants for the eye or a longer lived cosmetic filler than collagen alone for removal of wrinkles in anti-aging or rejuvenative applications (cosmetic surgery). In this regard, tests have demonstrated sub-cutaneous biocompatibility and stability after 30 days in rat), vi) delivery of genetically engineered cells, e.g. bone marrow stem cells that produce Factor VIII for delivery system in treatment of Haemophilia A; vii) nerve scaffolds, especially when fiber reinforced; and viii) implantation into other organs or tissues where scaffolding is needed - e.g. cardiac patches and cartilage replacements.

Although this application is largely directed to hydrogel material, it will be clear to the skilled worker that the material can be used as a base material to reconstruct skin, heart patches, encapsulation of genetically engineered cells for implantation (for example Factor 8 producing cells for Hemophilia) and nerve reconstruction. Bioactive factors, combinations of growth factors, peptides can be added to differentiate these base materials for these other tissue engineering/ regenerative medicine applications.

In one embodiment of the present invention, the material is pre-formed into an appropriate shape for tissue engineering purposes. In another embodiment the material is pre-formed as a full thickness artificial cornea or as a partial thickness material suitable for a cornea veneer.

In accordance with another embodiment of the present invention there is provided a device comprising a body including a material of the present invention that is effective in facilitating nerve growth through the body when the device is placed in an individual. For example when place in an eye of the individual, the material can facilitate nerve growth through the body, thereby permitting corneas receiving the device or devices to maintain their touch sensitivity. In the specific example in which the device is an ophthalmic device for placement in the eye of the individual, the body can be formed to have an optical power. Thus, the body may be understood to be a lens body. As discussed herein, the ophthalmic device may be configured, such as sized and shaped, to be a corneal onlay, a corneal inlay, or a full-thickness corneal implant. In certain embodiments, the ophthalmic device is a refractive error correcting device that does not have an optical power. For example, refractive error correcting devices in accordance with the present disclosure may be understood to be blanks that can be placed between a patient's corneal epithelium and Bowman's membrane, or in the patient's corneal stroma.

The material may also be used as a delivery system to deliver a bioactive agent to a particular region in a patient. Once within the body, the bioactive agent is released from the material, for example, through diffusion-controlled processes or, if the bioactive agent is covalently bound to the material, by enzymatic, chemical or physical cleavage from the material, and subsequent release by diffusion-controlled processes. Alternatively, the bioactive agent may exert its effects from within the material.

In one embodiment of the present invention, the bio-synthetic material is used as an artificial cornea. For this application, the material is pre-formed as a full thickness artificial cornea or as a partial thickness material suitable for a cornea veneer. In accordance with this embodiment, the hydrogel is designed to have a high optical transmission and low light scattering.

### Kits

The present invention also contemplates kits comprising the hydrogel material. The kits may comprise a "ready-made" form of the material or they may comprise the individual components required to make the material in appropriate proportions. The kit may optionally further comprise one or more bioactive agent. The kits may further comprise instructions for use, one or more suitable solvents, one or more instruments for assisting with the injection or placement of the final material composition within the body of an animal (such as a syringe, pipette, forceps, eye dropper or similar medically approved delivery vehicle), or a combination thereof. Individual components of the kit may be packaged in separate containers. The kit may further comprise a notice in the form prescribed by a governmental agency regulating the manufacture, use or sale of biological products, which notice reflects approval by the agency of the manufacture, use or sale for human or animal applications.

To gain a better understanding of the invention described herein, the following examples are set forth. It should be understood that these examples are for illustrative purposes only. Therefore, they should not limit the scope of this invention in any way.

### EXAMPLES

### EXAMPLE I - NiColl/MPC IPNs Hydrogels

**Materials.** Nippon collagen (swine skin); 0.625 M morpholinoethanesulfonic acid [MES, containing Aalizarin Red S pH indicator (6.5 mg/100ml water)]; 1-ethyl-3-(3-dimethyl aminopropyl) carbodiimide HCl (EDC), N-hydroxy-succinimide(NHS). MPC (2-methacryloyloxyethyl phosphorylcholine) was purchased from Biocompatibles International PLC (UK). NaOH solution (2N); PEG-diacrylate(Mw=575Da); and ammonium persulfate (APS) and N,N,N',N'-tetramethyl ethylene diamine (TEMED) were purchased from Sigma-Aldrich.

**Preparation of NiColl/MPC IPNs Hydrogels.** Initially, 0.3 ml of 13.7 wt% Nippon collagen solution and 0.1 ml of 0.625 M MES were mixed in two syringes connected with a plastic Tee in an ice-water bath. Subsequently, 12.9 mg of MPC (ratio of collagen to MPC was 4:1 w/w) was dissolved in 0.25 ml of MES, of which 0.2 ml was injected into the above mixture via a 100 $\mu$l microsyringe. Next, 4.6 $\mu$l of PEG-diacrylate, in weight ratio to MPC of 1:2, was injected using a 500 $\mu$l microsyringe. Unless otherwise stated, the ratio of PEG-diacrylate to MPC is fixed at 1:2. Then the solution was thoroughly mixed. Next, 25 $\mu$l of 2% APS and TEMED solution (in MES) was injected via a 100 $\mu$l microsyringe, followed by injection of 57 $\mu$l of EDC/NHS solution (in MES) in a molar ratio to collagen $NH_2$ of 3:3:1. The ratio of EDC to collagen was also kept constant for this study. NaOH (2N) was used to adjust the pH to about 5. The homogenous mixture was cast into a glass mold and incubated at room temperature with 100% humidity for 16 hours. The resulting molds were transferred to an incubator at 37°C for 5 hours, for port-curing. IPNs, denoted as NiColl/MPC IPNs hydrogels, with ratios of collagen to MPC of 1:1, 2:1 and 3:1 were prepared in using this same method.

As used in the present example, the terms NiColl/MPC IPN4-3, NiColl/MPC IPN3-3, NiColl/MPC IPN2-3 and NiColl/MPC IPN1-3 denote IPN gels from 13.7% Nippon collagen with ratios of collagen:MPC of 4/1, 3/1, 2/1 and 1/1, respectively.

### Characterisation

Refraction Index (RI). RIs of the samples were determined using a VEE GEE refractometer.

Optical Transmission. The optical transmission of samples was measured at wavelengths of white, 450, 500, 550, 600 and 650 nm, using a custom-designed instrument.

Mechanical Properties. The stress, break strain and moduli of samples was determined using an Instron electromechanical tester (Model 3340). The size of samples were 5 mm x 5mm x 0.5mm.

Water Content. The water contents of sample were calculated according to the following equation:

$$(W\text{-}W_0)/W\%$$

where $W_0$ and $W$ denote weights of dried and swollen samples, respectively.

**Results**

**Refractive index**

Table I lists the refraction index values of collagen hydrogels.

Table 1. RI of IPN samples

| Sample | NiColl/MPC IPN4-3 | NiColl/MPC IPN3-3 | NiColl/MPC IPN2-3 | NiColl/MPC IPN1-3 |
|---|---|---|---|---|
| Average RI* | 1.3448±0.0003 | 1.3472±0.0012 | 1.3464±0.0003 | 1.3525±0.0025 |

* denotes mean ± S.D.

**Optical transmission**

Table 2 summarizes the results of optical transmission.

Table 2. Optical Transmission

| Wavelength(nm) | White | 450 | 500 | 550 | 600 | 650 |
|---|---|---|---|---|---|---|
| Average Transmission (%) | | | | | | |
| NiColl/MPC IPN4-3 | 88.9±3.1 | 88.5±6.4 | 87.8±2.8 | 88.0±4.8 | 89.8±3.5 | 90.7±2.8 |
| NiColl/MPC IPN3-3 | 91.8±0.2 | 76.1±2,1 | 80.9±1.5 | 85.6±1.3 | 88.4±1.0 | 89.9±0.8 |
| NiColl/MPC IPN2-3 | 91.8±1.0 | 77.6±2.7 | 85.9±4.3 | 90.1±2.7 | 91.2±0.9 | 91.2±0.3 |
| NiColl/MPC IPN1-3 | 65.6±3.1 | 28.3±1.3 | 39.5±2.1 | 48.0±2.6 | 54.9±3.0 | 59.9±3.3 |
| NiColl/MPC IPN1-3 had become opaque. | | | | | | |

**Mechanical properties**

Table 3 presents the mechanical properties of IPN samples. NiColl/MPC IPN4-3 demonstrated a break stress as high as 360 KPa.

Table 3. Mechanical Properties

| Samples | NiColl/MPC IPN4-3 | NiColl/MPC IPN3-3 | NiColl/MPC IPN2-3 | NiColl/MPC IPN1-3 |
|---|---|---|---|---|
| Average Maximum Stress(KPa) | 361.0±122.3 | 262.2±70.3 | 155.9±46.8 | 213.0±80.8 |
| Average Break Stress (KPa) | 361.0±122.3 | 254.3±78.9 | 155.9±46.8 | 213.0±80.8 |
| Average Break Strain(%) | 22.80±2.68 | 17.49±2.79 | 24.83±1.36 | 24.80±4.06 |
| Average Modulus(MPa) | 3.338±0.883 | 2.536±0.187 | 1.344±0.491 | 1.571±0.425 |

**Equilibrated water content**

The equilibrated water contents of collagen gels were also measured (Table 4)

Table 4. Equilibrated Water Contents

| Samples | NiColl/MPC IPN4-3 | NiColl/MPC IPN3-3 | NiColl/MPC IPN2-3 | NiColl/MPC IPN1-3 |
|---|---|---|---|---|
| Water content (%) | 92.55±0.78 | 90.24±0.66 | 89.24±0.45 | 85.84±1.89 |

Biocompatibility Assays

*In vitro* biocompatibility assays indicated that the above described IPN hydrogels promoted epithelia growth well, and to a greater extent than controls (culture plate).

*In vivo* studies have demonstrated that coliagen/MCP containing IPN's support nerve growth.

Figure 19 shows *in vivo* confocal images of typical implants at six month post-operative compared with a set of typical untreated, contralateral, control corneas. Both (EDC/NHS) crosslinked recombinant human collagen and medical grade porcine collagen-MPC IPNs show that the re-growth of nerves into the stroma and sub-epithelial nerve network (arrows). This compares the IPN (right hand column) with crosslinked recombinant human collagen (centre column) and the left hand column untreated control. These results show that despite the significant synthetic component, the IPN is just as effective as the collagen only (i.e. completely natural polymer) in allowing nerve regeneration.

**References**

1. Schrader ME and Loeb GL (Eds), Modem approaches to wettabilty: theory and applications, Plenum Press, New York, pp 231-248 (1992).

2. Konno T, Hasuda H, Ishihara K, Ito Y. Photo-immobilization of a phospholipid polymer for surface modification. Biomaterials 2005, 26 :1381-1388.

3. Lewis AL, Crosslinkable coatings from phosphorylcholine-based polymers. Biomaterials 2001, 22:99-111.

## **EXAMPLE II - NiColl/MPC IPNs Hydrogels**

This study was performed to demonstrate the ability to alter the characteristics of IPNs prepared as in Example I by increasing the concentration of collagen solution from 13.7% to 20% at an EDC/Coll-$NH_2$ ratio of 1.5. Additionally, MES lacking a pH indicator was used.

**Materials.** Nippon collagen (swine skin); 0.625 M morpholinoethanesulfonic acid [MES, without pH indicator]; 1-ethyl-3-(3-dimethyl aminopropyl) carbodiimide HCl (EDC); N-hydroxy-succinimide(NHS). MPC was purchased from Biocompatibles Internationl PLC (UK). NaOH solution (2N); PEG-diacrylate (Mw=575Da); ammonium persulfate(APS); and N,N,N',N'-tetramethyl ethylene diamine (TEMED) were provided by Aldrich.

**Preparation of NiColl/MPC IPNs Hydrogels.**

Initially, 0.3 ml of 20.0 wt% Nippon collagen solution and 0.1 ml of MES (0.625 M) were mixed in two syringes connected with a plastic Tee in ice-water bath. Next, 12.9 mg of MPC (ratio of collagen to MPC, 4/1 w/w) was dissolved in 0.25 ml of MES, of which 0.2 ml was injected into the above mixture via a 100 μl microsyringe. Next, 4.6 μl of PEG-diacrylate in weight ratio to MPC of 1:2 was injected via a 500 μl microsyringe. The solution was thoroughly mixed. Next, 25 μl of 2% APS and TEMED solution (in MES) was injected via a 100 μl microsyringe, followed by injection of 86 μl of EDC/NHS solution (in MES) in molar ratio to Coll-$NH_2$ of 1.5:1.5:1. The homogenous mixture was cast into a glass mold or plastic molds (thickness 500 μm) and incubated at room temperature with 100% humidity for 16 hours. The molds were transferred to an incubator at 37°C for 5 hours, for post-curing.

As used herein, NiColl20/MPC IPN refers to IPNs made from 20% collagen solution.

**Property Measurement**

Refractive Index(RI). RIs of the samples were determined on a VEE GEE refractometer.

Optical Transmission. Transmission of hydrogel samples were measured at wavelengths of white, 450, 500, 550, 600 and 650 nm on a self-designed instrument.

Mechanical Properties. The tensile strength, elongation at break and elastic modulus of hydrogel samples were determined on an Instron electromechanical tester (Model 3340). The size of samples was 5 mm x 5mm x 0.5mm.

Water Content. The water content of hydrogel (WA) was calculated according to the following equation:

$$(W-W_0)/W \times 100\%$$

where $W_0$ and W denote weights of dried and swollen samples, respectively.

### Results

### Refractive index

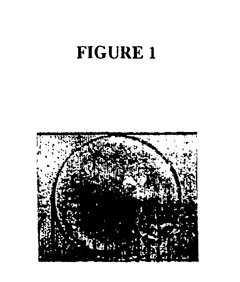

The IPN hydrogel from 20% solution was clear and uniform, (as shown in Figure 1). The refractive index was approximately 1.3519.

### Optical transmission

Table 5 summarizes the results of optical transmission.

Table 5. Optical properties

| Wavelength: | White | 450 | 500 | 550 | 600 | 650 |
|---|---|---|---|---|---|---|
| Transmission (%) | 87.9±2.2 | 81.1±2.2 | 83.1±2.2 | 83.2±2.2 | 84.9±2.2 | 86.6±2.2 |

### Mechanical properties

Table 2 lists the mechanical properties of gel. The tensile strength and modulus of NiColl20/MPC IPN are improved in comparison to those of the hydrogels prepared in Example 1. Importantly, this gel is flexible but hard (e.g., it cannot be broken using forceps).

Table 6. Mechanical properties

| Tensile strength (KPa) | Elongation at break(%) | Elastic Modulus(MPa) |
|---|---|---|
| 566.0±243.9 | 49.08±6.73 | 2093±1157 |

### Equilibrated water content

The equilibrated water content was 88.97%.

*In vitro* biocompatibility assays indicated that the NiColl20/MPC IPN hydrogel promoted epithelia growth well, and to a greater extent than controls (culture plate).

### EXAMPLE III - Novel Biosynthetic Materials for Vision Enhancing Ophthalmic

### Devices

The tissue-engirieered materials described in this example are essentially robust implantable materials with enhanced toughness and elasticity in comparison to materials previously known. Although they are collagen-based, they also incorporate biomimetic molecules such as chitosan that emulate natural extracellular matrix molecules (ECM) found within the human cornea while conferring significantly increased tensile strength. In addition, a hybrid cross-linking system was developed and used for stabilization of collagen/chitosan scaffolds to further enhance elasticity and toughness of the material. These enhanced materials were tested for mechanical, optical, and biological properties. Results suggest that scaffolds are tough, elastic, and superior to human eye bank corneas in optical clarity, and allow regeneration of corneal cells and nerves *in vitro.*

### Materials and Methods

The base material comprised a mixture of 10% (w/v) atelo-collagen type I and 3% (w/v) chitosan. Freeze dried porcine collagen powder that was obtained from Nippon Ham (Japan) was dissolved in cold water (sterile dd $H_2O$) and stirred at 4°C to give 10% (w/v) concentration. A 3% (w/v) chitosan solution was also prepared by dissolving chitosan powder (MW 40000 obtained from Fluka) in 0.2 N hydrochloric acid (HCl) and stirring at 4°C. The two solutions were then mixed in a syringe system at predetermined ratios to make a homogeneous blend prior to cross-linking. Various cross-linker agents (i.e., PEG dialdehyde and EDC/NHS) were used to develop interpenetrating networks (IPNs) with distinctive properties based on the type and concentration of cross-linkers and biomimetic component (see Table 7).

**Preparation of collagen-based corneal implants (IPN-I).**

Typically, 0.12 mL of 3% chitosan solution was added to 0.6 mL of a 10% collagen solution [chitosan:collagen at 0.5:1 molar ratio] in a Luer tip glass syringe. The composition was then mixed with 0.4 ml MES buffer using a Tefzel Tee-piece. The mixture was then mixed with EDC/NHS cross-linker agent [EDC:$NH_2$ (in collagen/chitosan) at 3:1 molar equivalent ratio and EDC:NHS at 1:1 molar equivalents ratio] in 0.35 ml MES buffer at about 0°C-4°C without air bubble entrapment. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

IPN-1 (EP10-2):

The collagen/chitosan blend was cross-linked using 1-ethyl-3-(3-dimethylaminopropyl) carbodiimide (EDC) and N-hydroxysuccinimide (NHS). The collagen/chitosan blend, and the EDC/NHS cross-linker were mixed together at an acidic pH of about 5 while preventing surges in pH using 2-(N-Morpholino)ethanesulfunic acid (MES) buffer. After sufficient mixing, portions of the mixed composition were placed in a mold, and were allowed to cure in the mold to form the network.

**Preparation of collagen-based corneal implants (IPN-II).**

0.02 ml of 3% chitosan solution was added to 0.6 ml of a 10% collagen solution [chitosan:collagen at 0.1:1 molar ratio] in a Luer tip glass syringe. The composition was then mixed with 0.4 ml MES buffer using a Tefzel Tee-piece. The mixture was then mixed with the hybrid cross-linker agent [PEG:$NH_2$ at 0.25:1 molar equivalent ratio, EDC:$NH_2$ at 4.5:1 molar equivalent ratio and EDC:NHS at 1:1 molar equivalents ratio] in 0.35 ml MES buffer at about 0°C-4°C without air bubble entrapment. The compositions were completely mixed by repeated pumping between the first and second syringes through the Tee.

Aliquots of each substantially homogeneous solution were immediately dispensed into 500 microns implant molds and cured first at room temperature for 16 hours, and then at 37°C for 16 hours, in 100% humidity environments at both temperatures. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

IPN-II (EP10-11):

The collagen/chitosan blend was cross-linked using a hybrid cross-linking system comprised of PEG-DiButylAldehyde (MW 4132 Da from Nektar Inc.) and 1-ethyl-3-(3-dimethylaminopropyl) carbodiimide (EDC) and N-hydroxy-succinimide (NHS). The collagen/chitosan blend, and the PEG-EDC/NHS hybrid cross-linker were mixed together at an acidic pH of about 5 while preventing surges in pH using 2-(N-Morpholino)ethanesulfonic acid (MES) buffer. After sufficient mixing, portions of the mixed composition were placed in a mold, and were allowed to cure in the mold to form IPN-II.

**Results**

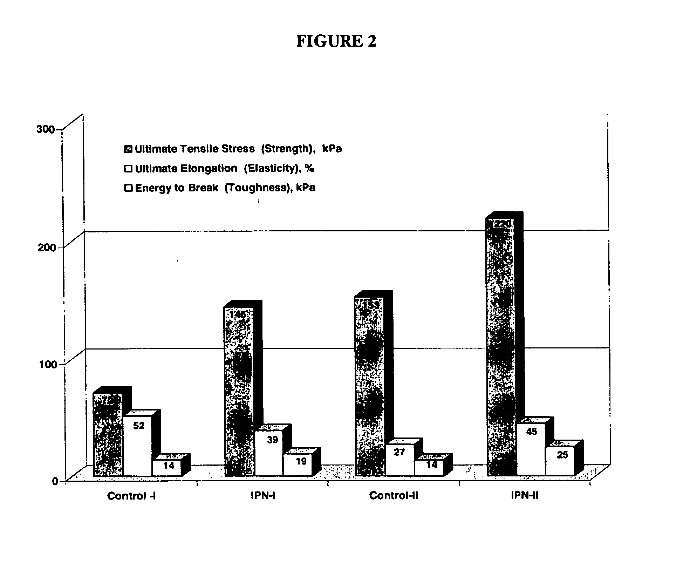

The IPN hydrogels prepared using hybrid cross-links have sufficient mechanical or structural properties to survive handling, implantation, which may include suturing, and post-installation wear and tear. As shown in Figure 2, they are mechanically stronger than the previously reported ophthalmic materials fabricated from 10% collagen cross-linked by EDC/NHS (Control I and Control II). For example, ultimate tensile strength, and toughness have been significantly enhanced when comparing IPN-I and IPN-II with control-I and control-II, respectively.

Generally, the enhancement of tensile strength, especially when induced by the increasing of cross-linking agent, is associated with the decrease of ultimate elongation (see the values for IPN-I compared to those for Control-I in Figure 2). This is probably a contribution from additional restraints to the mobility of the polymer network. This behavior was not observed for IPN-II hydrogel compared to Control-II and IPN-I. All mechanical properties including elasticity were enhanced for IPN-II hydrogel. This is most likely due to the formation of IP networks when chitosan and PEG are incorporated into the collagen scaffold. PEG can produce long cross-links as opposed to zero-length cross-links produced by EDC/NHS. This allows collagen molecules to move more freely resulting in more elastic scaffolds.

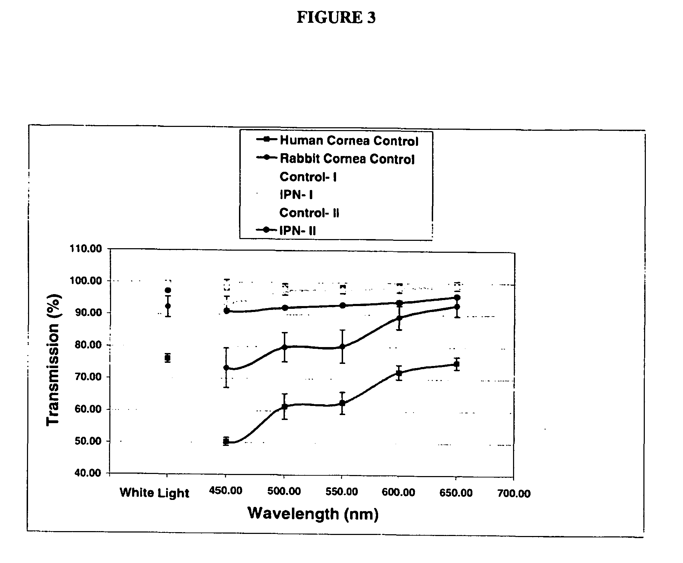

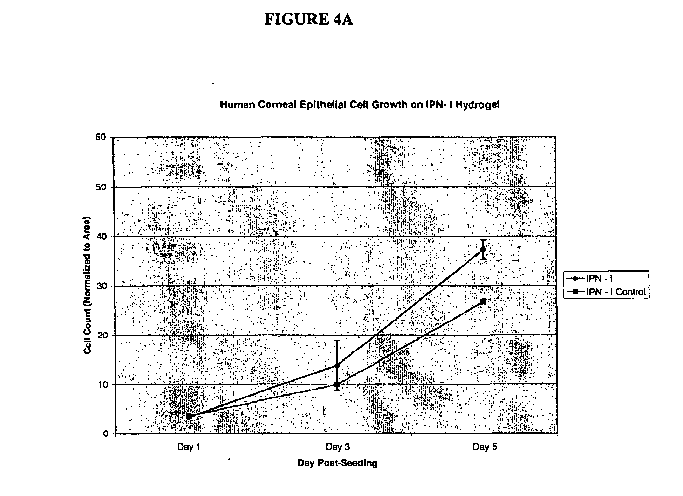

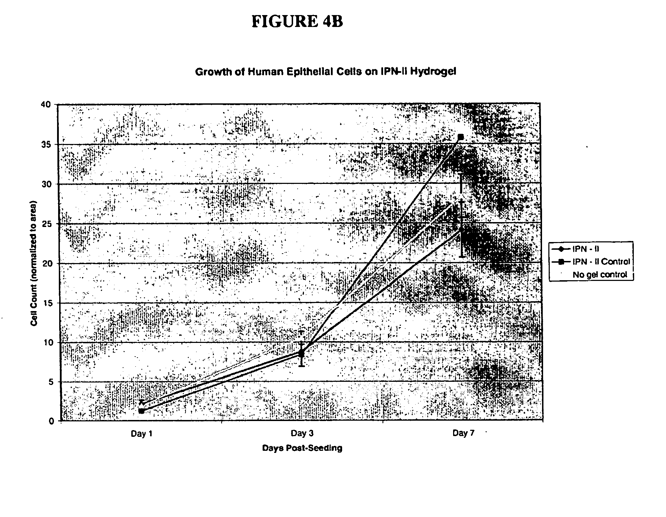

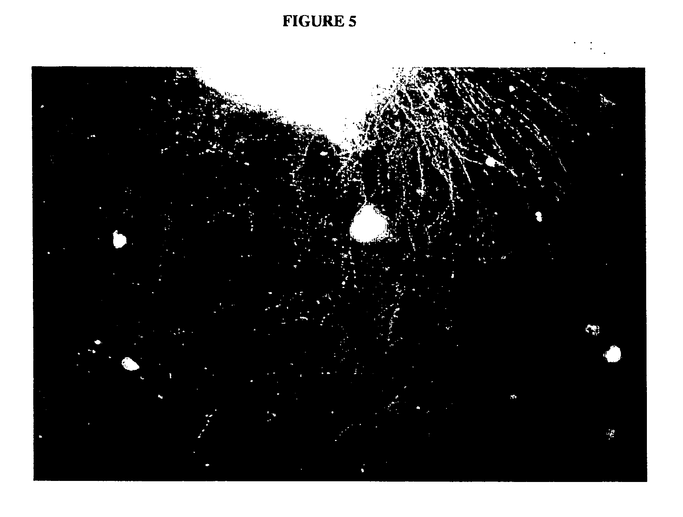

As summarized in Table 7 and depicted in Figure 3, the IPN hydrogels are optically transparent. They provide a desired optical clarity for visible light, optical transmission and light scattering equal to or better than those of healthy human cornea and rabbit cornea. These two materials are also non-cytotoxic. They allow regeneration of corneal epithelial cells as shown in Figure 4 (a) and (b). Figure 5 demonstrates nerve growth on IPN-II. The material seems to be nerve-friendly and allows nerves to grow over and likely through the hydrogel.

Table 7: Composition and performance of IPN materials and their controls

| Example or IPN No. | Collagen supplier, initial concentration | XL/Collagen Equiv. ratio | Chitosan: Collagen molar equivalent ratio | Maximum stress (kPa) | Maximum strain (%) | Toughness (kPa) | In vitro epi growth | In vitro nerve growth | % light transmission (white light) |
|---|---|---|---|---|---|---|---|---|---|
| IPN-I | Nippon Ham, 10% | EDC: NHS: $NH_2$ 3:3:1 | 0.5:1 | 145 | 39 | 19 | 7 | - | 99.4 |
| IPN-II | Nippon Ham, 10% | EDC: NHS: PEG: $NH_2$ 4-5:4.5:0.25:1 | 0.1:1 | 220 | 45 | 25 | 7 | Good | 97.3 |
| Control I | Nippon Ham, 10% | EDC: NHS: $NH_2$ 3:3:1 | 0 | 72 | 52 | 14 | 7 | - | 99 |
| Control II | Nippon Ham, 10% | EDC: NHS: $NH_2$ 6:6:1 | 0 | 153 | 27 | 14 | 7 | - | 99 |

With respect to EP10-11, it was found that the hydrogel's permeability to albumin and glucose was 1.67 x 10 (exp -7) and 2.8 x 10 (exp -6), respectively.

The EP10-11 hydrogel was also studied to demonstrate bio-compatibility/stability. Implants were placed under the skin of rats for 30 days to determine bio-compatibility and also stability. Some infiltration of immune cells was observed in one of 3 samples but samples were still intact after 30 days, showing stability.

Experiments were conducted to assess the growth of three bacterial species *(Staphylococcus aeureus, Streptococcus pneumonia* and *Pseudomonas aeurogenosa)*. The cornea matrix produced was a composite of the protein collagen and a synthetic N-isopropylacrylamide-based polymer molded to the same curvature and dimensions of a human cornea, with the optical clarity of a natural cornea. Each synthetic cornea was first sutured into a postmortem human cornea rim *in vitro* and the tensile strength and sutureability of the cornea was assessed. Different relative percentages of water, collagen and polymer were used to create 10 different cornea constructs. Each construct is reproduced in three sets of five and each set injected with 100 μl (0.1mL) of each of the noted bacteria. After the injection, the corneas are incubated at room temperature for 24-48 hours and growth of bacteria was assessed.

Results: Lower bacterial counts were observed in EP10-11 based cornea constructs than in human corneas from the eye bank.

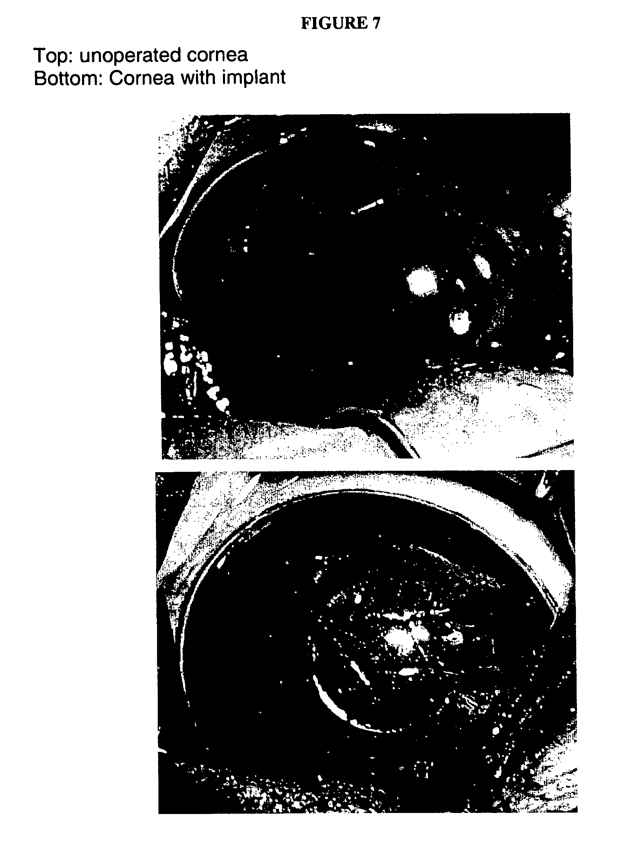

Figure 7 is a photograph of EP10-11 implanted into the cornea of a Yucatan mini-pig by lamellar keratoplasty (partial thickness graft). A 500 μm, 5 mm diameter graft was placed into the cornea of a pig (average thickness of a pig cornea is about 700-1000 μm).

## EXAMPLE IV - Collagen/PAA IPN Hydrogels

**Materials.** Nippon collagen(swine skin). 0.625 M morpholinoethanesulfonic acid [MES, containing Aalizarin Red S pH indicator (6.5 mg/100ml water)], 1-ethyl-3-(3-dimethyl aminopropyl) carbodiimide HCl (EDC), N-hydroxysuccinimide(NHS). Acrylic acid (AA) was purchased from Aldrich. PEG-diacrylate(Mw575), ammonium persulfate(APS) and N,N,N',N'-tetramethyl ethylene diamine (TEMED) were provided by Aldrich.

**Preparation of Collagen/PAA IPNs Hydrogels.** Firstly, 0.3 ml of 10.0 wt% Nippon collagen solution and 0.1 ml of MES(0.625 M) were mixed in two syringes connected with a plastic Tee in ice-water bath. Secondly, 30 μl of AA (ratio of collagen to AA, 1/1 w/w) was injected into the above mixture via a 100 μl microsyringe. Thirdly, 5.0 μl of PEG-diacrylate in weight ratio to AA of 1:5 was injected via a 50 μl microsyringe. Unless otherwise stated, the ratio of PEG-diacrylate to AA was fixed at 1:5. Then the solution was thoroughly mixed. Fourthly, 25 μl of 2% APS and TEMED solution (in MES) was injected via a 100 μl microsyringe, followed by injection of 57 μl of EDC/NHS solution(in MES) in molar ratio to collagen $NH_2$ of 6:6:1. The ratio of EDC to collagen was also kept constant for this series. The homogenous mixture was cast into a glass mold and left at room temperature with 100% humidity for 16 h. Then the molds were transferred into an incubator for post-curing at 37 °C for 5 h. The resulting hydrogel was robust and transparent.

## EXAMPLE V - Application of Materials for Drug, Bioactive Factor Delivery Systems to the Eye for the Cornea

Bio-synthetic materials have been developed that incorporate bioactive peptides or growth factors. These materials are useful primarily as corneal substitutes that have been shown to promote regeneration of corneal cells and re-growth of severed corneal nerves, in particular after incorporation of bioactive YIGSR (laminin) peptide (Li et al. 2003, 2005). Materials can also be adapted for delivery of growth factors (Klenker et al. 2005).

The objective was to develop materials that can be used for either one of two different modes for therapeutic delivery of bioactive factors to the cornea: 1) via implantable delivery systems (e.g. veneers, onlays, inlays, lamellar grafts); and 2) via therapeutic contact lenses.

Collagen corneal shields were developed in 1984 as corneal bandage lenses and are currently marketed for ocular surface protection following cataract and refractive surgery, penetrating keratoplasty, and traumatic epithelial defects. They are manufactured from porcine or bovine collagen and three different collagen shields are currently available with dissolution times of 12, 24, and 72 hours. The theoretical, experimental and clinical evidence supports a role for collagen corneal shields as a drug delivery device and in the promotion of epithelial and stromal healing.

However, drawbacks to these devices include their relatively short lifespan. The longest useable time for most is 72 hours. In addition, these are opaque in nature and visually occlusive. So such devices do not appear to have gained widespread usage.

Contact lens devices, in addition to optical indications, have a wide range of therapeutic applications in modem ophthalmology practice, such as, for relief of pain, mechanical protection and structural support, drug delivery and so on.

These fully synthetic hydrogel lens are not composed of natural biomaterials and therefore are not completely biocompatible. Complications associated with synthetic bandage contact lenses range from mild to severe. Examples include changes in corneal physiology, which can lead to epithelial, stromal, and endothelial compromise, lens deposition, allergic conjunctivitis, giant papillary conjunctivitis, peripheral infiltrates, microbial keratitis, and neovascularization.

A highly biocompatible, suitable for extended wear therapeutic contact lens that may be also able to load drugs is therefore highly desirable. Suitable lenses can be made based on two main groups of materials.

**• Chitosan-synthetic polymer IPN hydrogel lens**

Chitosan, a primary component of the exoskeleton of crustaceans, has recently received a great deal of interest for medical and pharmaceutical applications. It has been reported to promote wound-healing and also has bacteriostatic effects. About 20 years ago, chitosan was suggested as a good material for contact lens manufacturing, but this has not met with success because chitosan is not soluble in neutral water and chitosan gel does not have a good mechanical strength.

As described herein, chitosan and chitosan derivatives were used in the development of materials for use as corneal substitutes for transplantation. These materials have were found to have good mechanical strength and elasticity for hydrogels. They were also tested in *vitro* and subcutaneously in animals and are currently undergoing testing in rodent and pig models in LKP surgeries.

**Therapeutic Contact Lens Device Development**

In accordance with one aspect of the invention, a highly biocompatible bandage contact lens for therapeutic use, has the following features:

1. Promote wound-healing
2. Bacteriostatic
3. Able to load various drugs, such as NGF-beta for neurotrophic keratitis treatment.
4. Extended wear to 2-3 weeks or even longer.
5. Transparent (>90% light transmission, <3% scatter) - allows vision

This was achieved using bio-synthetic polymers that combine the biological properties of chitosan with the mechanical properties of synthetic hydrogels by forming interpenetrating networks. The properties of these materials can be enhanced by addition of collagen. Collagen refers to the glycoprotein that is from extracted animal sources (e.g. porcine ateocollagen, or recombinant human collagen such as type I and type III collagen available from Fibrogen). In addition, various bioactive factors (e.g., peptides, growth factors or drugs) can be incorporated using methods previously developed.

<u>Manufacturing Method</u>

Water soluble chitosan and partially carboxymethylated chitosan was used. A synthetic monomer or crosslinker e.g., acrylic acid, PEG-diacrylated, methacrylic acid and vinyl pyrrolidone etc., is used.

**• Collagen-based bio-synthetic lenses**

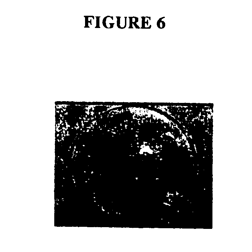

A range of collagen-synthetic copolymeric IPNs having 88.9 % water content, a refractive index of 1.35, and about 88 % light transmission (for 500 $\mu$m thick samples; see Figure 6) have now been developed.

Mechanical properties achieved for these hydrogels are as follows:

| Tensile strength (KPa) | Elongation at break(%) | Elastic Modulus(MPa) |
|---|---|---|
| 566.0±243.9 | 49.08±6.73 | 2093±1157 |

It is possible to further improve the characteristics of these hydrogels, for example by increasing the refractive index, increasing light transmission, or by improving mechanical strength. To improve mechanical strength, higher concentration of components such as collagen and the synthetic monomer can be used to form the IPN or the hydrogel can be immersed into a solution containing one or more monomers to form one or more additional polymeric networks, so the strength of the hydrogel should be enhanced. The materials have been found to be biocompatible *in vitro* and *in vivo* as subcutaneous implants; and can be implanted as corneal substitutes and as a base material for incorporation of bioactive factors.

Using the materials described herein, it is possible to manufacture contact lenses that are biocompatitible and that promote wound-healing and/or are bacteriostatic. Such contact lenses can include various loaded drugs, bioactive peptides and/or growth factors such as NGF for neurotrophic keratitis treatment. Further, collagen can be incorporated into this chitosan-synthetic lens to enhance biocompatibility and drug loading. The manufactured contact lenses can optionally be suitable for extended wear to 2-3 weeks or longer.

## EYMAPLE VI - ADHESIVES FOR ANTERIOR AND POSTERIOR COMPARTMENTS OF THE EYE

To repair penetrating corneal wounds, such as corneal breaks, suturing has been a successful method. However, suturing has some disadvantages, such as prolonged surgical time and the requirement of surgical skills. Suturing may also cause significant topographic distortion and high levels of astigmatisms. Loose sutures may harbor bacteria and cause inflammation and tissue necrosis. Additionally, sutures can cause significant discomfort. In addition, non-biodegradable sutures need to remove, which extends patient's follow up.

Adhesives that have also found similar application in the various types of tissue adhesives can be subdivided into synthetic adhesives (e.g. cyanoacrylate derivatives) and biologic adhesives (e.g. fibrin-based adhesives). Cyanoacrylate derivatives are compounds with very high tensile strength that rapidly polymerize on contact with basic substances such as water or blood to form a strong bond. Because they are synthetic and non-biodegradable, they are usually used on an external surface and may induce an inflammatory foreign body reaction, including neovascularization and tissue necrosis. In ophthalmology, cyanoacrylate derivatives have mainly been used in the management of corneal perforations and severe thinning, although they have been tried in various other ophthalmic surgeries.

Fibrin-based adhesives, by contrast, have a lower tensile strength and slower polymerization, however, being biologic and biodegradable, they may be used under a superficial covering layer (e.g., conjunctiva, amniotic membrane) and induce minimal inflammation. These adhesives have been used in ophthalmology to treat corneal thinning and perforations, ocular surface disorders, and glaucoma. More recently, fibrin-based adhesives have been used to perform sutureless lamellar keratoplasty and to attach amniotic membrane to bare sclera. Unfortunately, because fibrin glues use human thrombin, this blood product still carries a risk of infection and viral transmission from a contaminated donor pool. In addition, the preparation and application of fibrin-based glues is significantly more complex than that of cyanoacrylate glues.

Natural biopolymers or their derivatives can be used to fabricate biocompatible, biodegradable, high tensile strength, non-toxic and safe tissue adhesives. A two-component glue in which the gelling speed can be adjusted by modifications of pendant groups can be used. Drugs, bioactive peptides and growth factors can also be incorporated into the gelling system for sustained release. In addition, because gelling rates can be controlled as well as viscosity, this system can also be used for delivery of stem and progenitor cells.

The tissue adhesive has two components 1) oxidized chondroitin sulfate and 2) and water soluble chitosan. Upon mixing, these two components will form a glue or gel via reactions between the aldehyde groups on chondroitin sulfate and the amine groups on chitosan. The gelling speed can be adjusted by tuning the oxidization degree of the adjacent hydroxyl groups of chondroitin sulfate.

Drugs, growth factors (e.g. NGF-beta) and bioactive peptides (e.g. combinations of laminin, fibronectin, syngistic substance P and IGF-like peptides) can also be incorporated into the gelling system for sustained release.

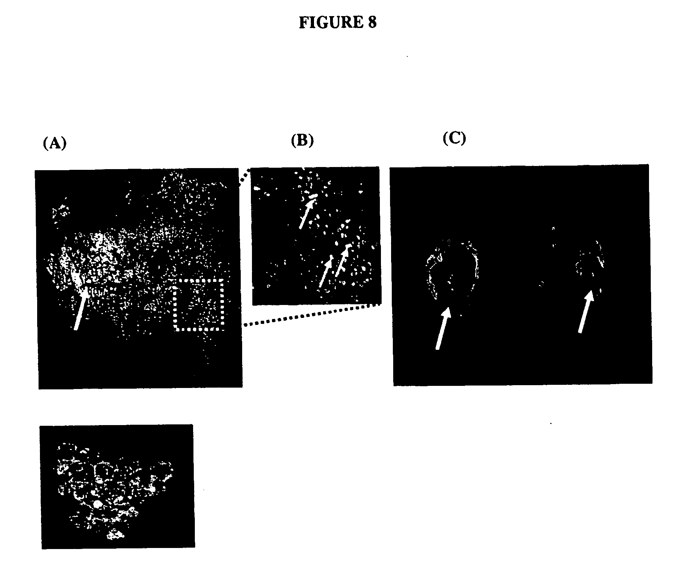

The chondroitin sulphate-based material can be used to deliver endothelial progenitor cells (EPCs) into a muscle test system and obtained incorporation of labelled EPCs (labelled green in following figure) into blood vessels via angiogenesis, as seen in Figure 8. Figure 8 is depicts a chondroitin sulphate-based material to deliver endothelial progenitor cells (EPCs) into a muscle test system and obtaining incorporation of labeled EPCs (labeled green in following figure) into blood vessels via angiogenesis, (A) Injection site (arrow) of EPCs (green labeled) in skeletal muscle from rat ischemic hindlimb, (B) Magnified image of EPCs (arrows) migrating from injected matrix into the tissue, (C) EPCs were observed within blood vessel structures (arrows).

## References

Klenkler B.J., Griffith M., Becerril C., West-Mays J., Sheardown H. (2005) EGF-grafted PDMS surfaces in artificial cornea applications. Biomaterials 26: 7286-7296.

Li, F., Carlsson, D.J., Lohmann, C.P., Suuronen, E.J., Vascotto, S., Kobuch, K., Sheardown, H., Munger, M. and Griffith, M. (2003) Cellular and nerve regeneration within a biosynthetic extracellular matrix: corneal implantation. Proc. Natl. Acad. Sci. USA 100: 15346-15351.

Li, F., Griffith, M., Li, Z., Tanodekaew, S., Sheardown, H., Hakim, M. and Carlsson, D.J. (2005) Recruitment of multiple cell lines by collagen-synthetic copolymer matrices in corneal regeneration. Biomaterials 26:3039-104.

**EXAMPLE VII - Recombinant Human Type III Collaeen-MPC IPN with 12.1 % w/w collagen**

Firstly, 0.3 ml of 12.1 wt% Type III collagen solution and 0.1 ml of MES(0.625 M) were mixed in two syringes connected with a plastic Tee in ice-water bath. Secondly, 50 mg of MPC (ratio of collagen to MPC, 1/1 w/w) was dissolved in 0.138 ml of MES, of which 0.1 ml was injected into the above mixture via a 100 $\mu$l microsyringe. Thirdly, 16 $\mu$l of PEG-diacrylate (Mn=575) in weight ratio to MPC of 1:2 was injected via a 100 $\mu$l microsyringe. Then the solution was thoroughly mixed. Fourthly, 25 $\mu$l of 2% APS and TEMED solution(in MES) was injected via a 100 $\mu$l microsyringe, followed by injection of 57 $\mu$l of EDC/NHS solution(in MES) in molar ratio to Coll-NH$_2$ of 3:3:1. The homogenous mixture was cast into a glass mold or plastic molds (thickness 500 $\mu$m) and left at room temperature with 100% humidity for 16 h. Then the molds were transferred into an incubator for post-curing at 37 °C for 5 h.

The resultant hydrogel was robust and optically clear, whereas Nippon collagen/MPC collagen prepared in the same condition was opaque. The merit of rhc Type III is its ability to remain transparent in wide range of pH and crosslinker content.

**EXAMPLE VIII - COLLAGEN OR COLLAGEN HYDROGEL**

As in Example III, the ophthalmic materials described in this section are essentially robust implantable materials with enhanced toughness and elasticity in comparison to materials previously known. Although they are collagen-based, they also incorporate biomimetic molecules such as chitosan that emulate natural extracellular matrix molecules (ECM) found within the human cornea while conferring significantly increased tensile strength. In addition, a cross-linking system was developed and used for stabilization of collagen and collagen/chitosan scaffolds to further enhance elasticity and toughness of the material. These enhanced materials were tested for mechanical, optical, and biological properties. Results suggest that scaffolds are tough, elastic, and superior to human eye bank corneas in optical clarity, and allowed regeneration of corneal cells and nerves *in vitro.*

**Materials and general methods**

The base material comprised a mixture of 10% (w/v) atelo-collagen type I and 3% (w/v) chitosan. Freeze dried porcine collagen powder that was obtained from Nippon Ham (Japan) was dissolved in cold water (sterile dd H$_2$O) and stirred at 4°C to give 10% (w/v) concentration. A 3% (w/v) chitosan solution was also prepared by dissolving chitosan powder (MW 400000 obtained from Fluka) in 0.2 N hydrochloric acid (HCl) and stirring at 4°C. The two solutions were then mixed in a syringe system at predetermined ratios to make a homogeneous blend prior to cross-linking. Various cross-linker agents such as PEG-dibutylaldehyde (MW 3400 Da from Nektar Inc.) and 1-ethyl-3-(3-dimethylaminopropyl) carbodiimide (EDC) and N-hydroxysuccinimide (NHS) were used to develop interpenetrating networks with distinctive properties based on the type and concentration of cross-linkers and biomimetic component (see Table 8).

**EXAMPLE IX - Fabrication of collagen hydrogel crosslinked by EDC/NHS and PEG-dibutyaldehyde(HPN-3)**

0.6 mL of a 10% collagen solution in a Luer tip glass syringe was mixed with 0.4 ml MES buffer using a Tefzel Tee-piece. The collagen blend was cross-linked using a cross-linking system comprised of PEG-dibutylaldehyde and EDC/NHS [PEG:NH$_2$ at 0.36:1 molar equivalent ratio, EDC:NH$_2$ at 5.4:1 molar equivalent ratio and EDC:NHS at 1:1 molar equivalents ratio] in 0.35 ml MES buffer at about 0°C-4°C without air bubble entrapment. The pH of 5 was maintained during the cross-linking reaction using MES buffer. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures to form HPN-3. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

**EXAMPLE X - Fabrication of collagen-chitosan hydrogel crosslinked with EDC/NHS and PEG-dibutyaldehvde (HPN-4)**

0.036 mL of 3% chitosan solution was added to 0.6 mL of a 15% collagen solution [chitosan:collagen at 0.01:1 molar ratio] in a Luer tip glass syringe. The composition was then mixed with 0.4 ml MES buffer using a Tefzel Tee-piece. The collagen/chitosan blend was cross-linked using a hybrid cross-linking system comprised of PEG-dibutylaldehyde and EDC/NHS [PEG:NH$_2$ at 0.3:1 molar equivalent ratio, EDC:NH$_2$ at 4.5:1 molar equivalent ratio and EDC: NHS at 1:1 molar equivalents ratio] in 0.35 ml MES buffer at about 0°C-4°C without air bubble entrapment. The pH of 5 was maintained during the cross-linking reaction using MES buffer. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures to form HPN-4. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

## EXAMPLE XI: Fabrication of collagen hydrogel crosslinked by EDC/NHS and PEG-dibutyaldehyde (HPN-5)

0.6 mL of a 15% collagen solution in a Luer tip glass syringe was mixed with 0.4 ml MES buffer using a Tefzel Tee-piece. The collagen blend was cross-linked using a hybrid cross-linking system comprised of PEG-dibutylaldehyde and EDC/NHS [PEG:$NH_2$ at 0.36:1 molar equivalent ratio, EDC:$NH_2$ at 5.4:1 molar equivalent ratio and EDC:NHS at 1: 1 molar equivalents ratio] in 0.35 ml MES buffer at about 0°C-4°C without air bubble entrapment. The pH of 5 was maintained during the cross-linking reaction using MES buffer. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures to form HPN-5. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

## EXAMPLE XII: Fabrication of collagen hydrogel crosslinked by PEG-dibutyaldehyde (HPN-6)

0.6 mL of a 20% collagen solution in a Luer tip glass syringe was mixed with 0.4 ml MES buffer using a Tefzel Tee-piece. The collagen blend was cross-linked using PEG-dibutylaldehyde [PEG:$NH_2$ at 1:1 molar equivalent ratio] in 0.35 ml MES buffer at about 0°C-4°C without air bubble entrapment. The pH of 5 was maintained during the cross-linking reaction using MES buffer. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures to form HPN-6. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproducts.

## EXAMPLE XIII: Fabrication of collagen hydrogel crosslinked by PEG-dibutyaldehyde (HPN-7)

Typically, 0.6 mL of a 20% collagen solution in a Luer tip glass syringe was mixed with 0.4 ml MES buffer using a Tefzel Tee-piece. The collagen blend was cross-linked using PEG-dibutylaldehyde [PEG:$NH_2$ at 2:1 molar equivalent ratio] in 0.35 ml MES buffer at about 0°C-4°C without air bubble entrapment. The pH of 5 was maintained during the cross-linking reaction using MES buffer. The compositions were completely mixed by repeated pumping between the first and second syringes through the Tee.

Aliquots of each substantially homogeneous solution were immediately dispensed into 500 micron implant molds and cured first at room temperature for 16 hours, and then at 37°C for 16 hours, in 100% humidity environments at both temperatures to form HPN-7. Each final implant sample was carefully separated from its mold after immersion in phosphate buffered saline (PBS) for 2 hours. Finally, the cross-linked implant hydrogels were immersed in PBS solution (0.5% in PBS, containing 1% chloroform) at 20°C to terminate any reactive residues and to extract out reaction byproduct.

Table 8. Composition and performance of HPN materials and their controls

| Formulation code | Collagen supplier, initial concentration | XL/Collagen Equiv. ratio | Chitosan:Collagen molar equivalent ratio | Maximum stress (kPa) | Maximum strain (%) | Toughness (kPa) | In vitro epi growth | % light transmission (white light) |
|---|---|---|---|---|---|---|---|---|
| HPN-3 | Nippon Ham. 10% | EDC: NHS: PEG: $NH_2$ 5.4: 5.4:0.4:1 | 0 | 276 ± 25 | 40 ± 6.1 | 35 ± 6 | Good | 98 ± 0.9 |
| HPN-4 | Nippon Ham. 15% | EDC: NHS: PEG: $NH_2$ 45: 4.5:03:1 | 0.01:1 | 69 ± 12 | 105 ± 9 | 30 ± 2 | Moderate to Good | 90.4 ± 0.5 |
| HPN-5 | Nippon Ham. 15% | EDC: NHS: PEG: $NH_2$ 5.4 : 5.4: 0.4 :1 | 0 | 216 ± 45 | 71.5 ± 6.7 | 65 ± 7 | Good | 51.4 ± 1.9 |
| HPN-6 | Nippon Ham, 20% | PEG: $NH_2$ 1:1 | 0 | - | - | - | Degradable | 97.5 ± 1.0 |
| HPN-7 | Nippon Ham. 20% | PEG: $NH_2$ 2:1 | 0 | - | - | - | Degradable | 98.6 ± 0.4 |
| Control 1 | Nippon Ham. 10% | EDC: NHS: $NH_2$ 3:3:1 | 0 | 72 ± 9 | 52 ± 2.8 | 13.9 ± 2 | Excellent | 99 ± 0.2 |
| Control 2 | Nippon Ham. 10% | EDC: NHS: $NH_2$ 6:6:1 | 0 | 153 ± 23 | 27 ± 2.7 | 13.6 ± 3 | Excellent | 99 ± 0.5 |

**EXAMPLE XIV: Collagen matrix fabricated from recombinant human Type I collagen and EDC/NHS**

An aliquot of recombinant human collagen type I solution (12.7% w/w) was loaded into a syringe mixing system free of air bubbles Calculated volumes of EDC, and NHS (both at 10% w/v, EDC:Collagen-NH2 ratio=0.4:1; EDC:NHS ratio=1:1) were added through the septum from a second syringe and again thoroughly mixed at 0°C. The final solution was immediately dispensed onto a glass plate to form a flat film. The flat film was cured at 100% humidity (at 21 °C for 24 h and then at 37 °C for 24 h). The films were washed 3 times with fresh PBS and then stored in PBS containing I % chloroform to maintain sterility. The gel with other ratios of EDC/Coll-NH$_2$ were prepared in the same fashion.

Table 9 Mechanical properties of type I recombinant collagen hydrogel

| EDC/Coll-NH$_2$ | 0.3 | | 0.4 | | 0.5 | | 0.6 | | 0.7 | |
|---|---|---|---|---|---|---|---|---|---|---|
| | Avg. | Std. Dev. | Avg. | Std. Dev. | Avg. | Std. Dev. | Avg. | Std. Dev. | Avg. | Std. Dev. |
| Max Load, kN | 0.0018 | 0.0004 | 0.003 | 0.0001 | 0.0025 | 0.0008 | 0.0024 | 0.0005 | 0.0028 | 0.0004 |
| Max Stress, Mpa | 0.7346 | 0.1436 | 1.19 | 0.057 | 9.849 | 0.3081 | 0.9681 | 0.1839 | 1.125 | 0.1771 |
| Modulus, Mpa | 9.703 | 1.812 | 15.17 | 1.84 | 15.45 | 2.83 | 12.93 | 0.34 | 12.21 | 1.23 |
| Break strain% | 18.67 | 0.86 | 18.89 | 2.69 | 15.41 | 1.2 | 13.56 | 1.71 | 15.17 | 1.73 |
| Break Strain | 0.7496 | 0.1612 | 1.19 | 0.057 | 0.9849 | 0.3081 | 0.9681 | 0.1839 | 1.125 | 0.1771 |
| Toughness | 0.037 | 0.0079 | 0.0618 | 0.0133 | 0.0397 | 0.014 | 0.455 | 0.0132 | 0.0609 | 0.0176 |

**EXAMPLE XV: Collagen matrix fabricated from recombinant human Type III collagen and EDC/NHS**

An aliquot of recombinant human collagen type III solution (12.7% w/w) was loaded into a syringe mixing system free of air bubbles Calculated volumes of EDC, and NHS (both at 10% wt/vol, EDC:Collagen-NH2 ratio =0.4:1; EDC: NHS ratio =1:1) were added through the septum from a second syringe and again thoroughly mixed at 0°C. The final solution was immediately dispensed onto a glass plate to form a flat film. The flat film was cured at 100% humidity (at 21 °C for 24 h and then at 37 °C for 24 h). The films were washed 3 times with fresh PBS and then stored in PBS containing 1 % chloroform to maintain sterility. The gel with other ratios of EDC/Coll-NH$_2$ were prepared in the same fashion.

Table 10 Mechanical properties of type III collagen gels

| EDC/Coll-NH$_2$ | 0.3 | | 0.4 | | 0.5 | | 0.6 | | 0.7 | |
|---|---|---|---|---|---|---|---|---|---|---|
| | Avg. | Std. Dev. | Avg. | Std. Dev. | Avg. | Std. Dev. | Avg. | Std. Dev. | Avg. | Std. Dev. |
| Max Load, kN | 0.0054 | 0.0018 | 0.0043 | 0.2054 | 0.0035 | 0.0024 | 0.004 | 0.0005 | 0.0052 | 0.0018 |
| Max Stress, Mpa | 2.173 | 0.725 | 1.7 | 0.2054 | 1.385 | 0.8339 | 1.588 | 0.196 | 2.098 | 0.702 |
| Modulus, Mpa | 22.39 | 5.64 | 20.26 | 2.04 | 16.6 | 4.5 | 16.48 | 1.41 | 17.91 | 1.44 |
| Break strain% | 16.33 | 2.89 | 13.88 | 0.69 | 14.43 | 3.18 | 18.17 | 4.64 | 18.54 | 4.1 |
| Break strain | 2.173 | 0.725 | 1.7 | 0.2054 | 1.385 | 0.8339 | 1.293 | 0.707 | 2.098 | 0.702 |
| Toughness | 0.1422 | 0.0761 | 0.0881 | 0.0049 | 0.0867 | 0.0773 | 0.1142 | 0.0261 | 0.1435 | 0.0757 |

## EXAMPLE XVI: Cornea matrix fabricated from recombinant human type I and type III dual collagen and EDC/NHS

An aliquot of recombinant human collagen type I solution (12.7% w/w) was loaded into a syringe mixing system free of air bubbles and weighed. An aliquot of recombinant human collagen type III solution (12.7% w/w) of equal weight was loaded into the same syringe mixing system to give a 50/50 wt/wt % solution of collagen type I and type III solution in the syringe mixing system. Calculated volumes of EDC, and NHS (both at 10% (w/v), EDC:NH2 ratio =0.4:1; EDC: NHS ratio =1:1) were added through the septum from a second syringe and again thoroughly mixed at 0°C. The final solution was immediately dispensed onto a glass plate to form a flat film. The flat film was cured at 100% humidity (at 21 °C for 24 h and then at 37 °C for 24 h). The films were washed 3 times with fresh PBS and then stored in PBS containing 1 % chloroform to maintain sterility. The water content of the final gel was 93.3%.

Table 11 Mechanical properties of type I-III gel at 1:1 w/w ratio

| EDC/Coll-NH$_2$ | EDC = .4 | |
|---|---|---|
| | Avg. | Std. Dev. |
| Max Load, kN | 0.0039 | 0.0004 |
| Max Stress, Mpa | 1.555 | 0.167 |
| Modulus, Mpa | 16.11 | 2.74 |
| Break strain% | 16.17 | 1.77 |
| Break Strain | 1.555 | 0.167 |
| Toughness | 0.0849 | 0.0042 |

Table 12 Collagen matrix denature temperature results by DSC

| EDC/Coll-NH$_2$ | Type I, $T_{onset}$ °C | Type I, $T_{max}$ °C | Type III, $T_{onset}$ °C | Type III, $T_{max}$ °C |
|---|---|---|---|---|
| 0.3 | 46.15 | 47.9 | 55.3 | 57.32 |
| 0.4 | 56.01 | 58.58 | 58.05 | 60.59 |
| 0.5 | 55.22 | 56.77 | 58.63 | 61.72 |
| 0.6 | 55.87 | 58.38 | | |
| 0.7 | 52.4 | | | |
| 12.7% type 1 solution | 37.65 | 45.52 | | |

Table 13 Water content of final collagen matrix

| EDC/Coll-NH$_2$ | Type III, Avg. % | Type III, Std. Dev | Type I, Avg.% | type I, Std. Dev |
|---|---|---|---|---|
| 0.3 | 91.4 | 3 | 85.9 | 0.96 |
| 0.4 | 89.9 | 2.7 | 89.6 | 1.8 |
| 0.5 | 90.5 | 0.5 | 89.9 | 0.61 |
| 0.6 | 88.3 | 1.8 | | |
| 0.7 | 89.6 | 4.7 | | |

## EXAMPLE XVII: Collagen-synthetic interpenetrating polymeric networks

## Materials

Nippon collagen (swine skin); 0.625 M morpholinoethanesulfonic acid [MES, containing Aalizarin Red S pH indicator (6.5 mg/100ml water)]; 1-ethyl-3-(3-dimethyl aminopropyl) carbodiimide HCl (EDC), N-hydroxy-succinimide (NHS). NaOH solution (2N); PEG-diacrylate(Mw575); and ammonium persulfate (APS) and N,N,N',N'-tetramethyl ethylene diamine (TEMED) were purchased from Sigma-Aldrich.

N,N-dimethylacrylamide (99%) was purchased from Sigma-Aldrich. Inhibitor was removed by using an inhibitor remover purchased form Sigma-Aldrich.

Preparation of Coll-DMA IPNs Hydrogels.

Firstly, 0.3 ml of 13.7 wt% Nippon collagen solution and 0.3 ml of MES (0.625 M) were mixed in two syringes connected with a plastic Tee in ice-water bath. Secondly, 14.2 ml of DMA (ratio of collagen to DMA, 3/1 w/w) was injected into the above mixture via a 50 $\mu$l microsyringe. Thirdly, 6.14 $\mu$l of PEG-diacrylate (Mn=575) in weight ratio to DMA of 1:2 was injected via a 100 $\mu$l microsyringe. Unless otherwise stated, the ratio of PEG-diacrylate to DMA is fixed at 1:2. Then the solution was thoroughly mixed. Fourthly, 1 % APS and 1 % TEMED solution (in 25 ul MES) relative to DMA was injected via a 100 $\mu$l microsyringe, followed by injection of 57 $\mu$l of EDC/NHS solution(in MES) in molar ratio of EDC:NHS:Coll-NH$_2$ =3:3:1. The ratio of EDC to collagen was also kept constant for this series. The homogenous mixture was cast into a glass mold and left at room temperature with 100% humidity for 16 h. Then the molds were transferred into an incubator for post-curing at 37 °C for 5 h. Coll-DMA IPNs hydrogels with ratios of collagen to DMA, 1:1, 2:1 and 4:1 were prepared in the same method.

Properties of hydrogels

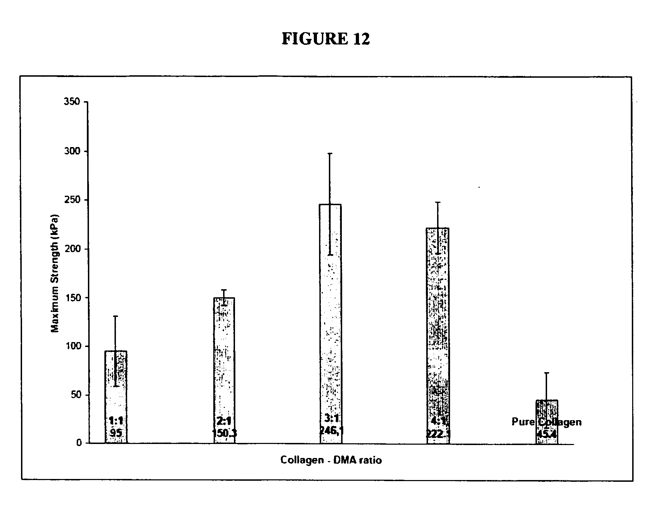

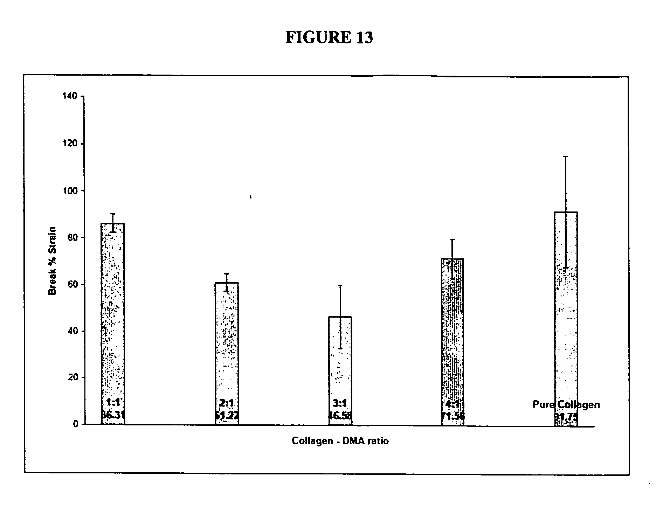

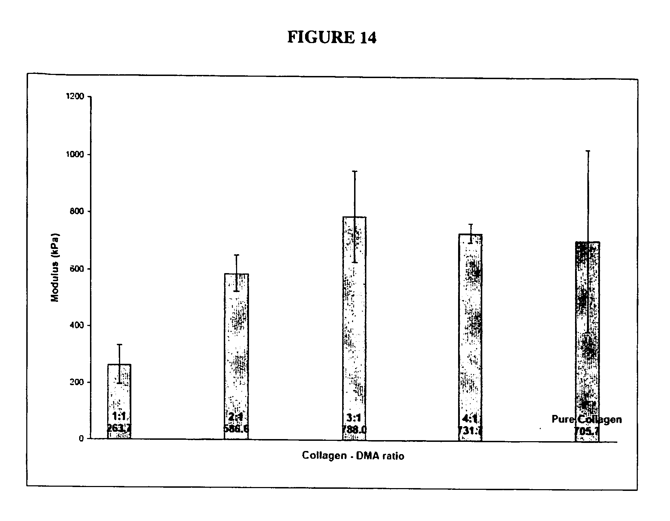

Figure 12, Figure 13 and Figure 14 show the tensile, strain and modulus results of all gels from DMA: collagen =1:1, 1:2, 1:3 and 1:4 (w/w).

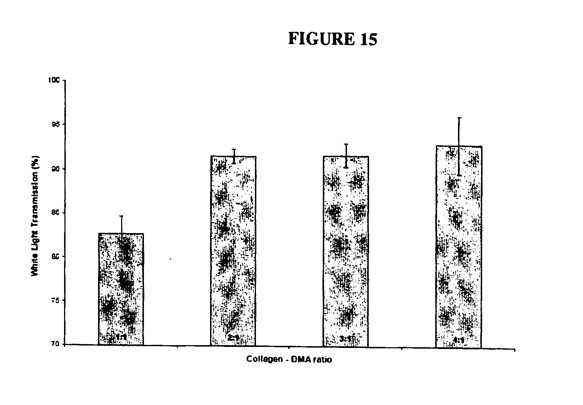

Figure 15 shows the white light transmission results of Collagen-DMA hydrogels. For all ratios, except 1:1 gel, the white light transmission is over 90%, comparable or superior to that of human cornea.

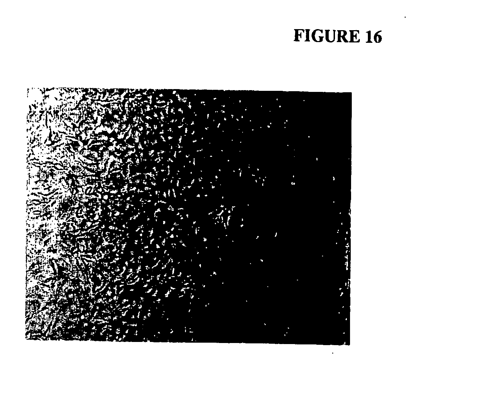

Collagen-DMA hydrogels are very biocompatible and supporting epithelium over-growth (see Figure 16). The epithelium cells became confluent in 3 days of culture.

**EXAMPLE XVIII: Opthalmic Devices Containing Bio-Active Agents**

Hydrogel materials incorporating bioactive agents such as anti-bacterial, anti-viral agents or growth factors such as neutrophic factors, constitute advanced devices that may be useful for, for example, cornea transplantation or as a therapeutic lens for drug delivery or wound healing. Examples of antibacterial peptides to be incorporated into an IPN hydrogel include, but are not limited to:

peptide #1:

Acid-CGSGSGGGZZQOZGOOZOOZGOOZGY-NH$_2$

Peptide #2:

Acid-GZZQOZGOOZOOZGOOZGYGGSGSGC-NH$_2$

These peptides contain the basic peptide sequences reported by Giangaspero et al (Giangaspero, A., Sandri, L., and Tossi, A. , Amphipathic alpha helical antimicrobial peptides. A systematic study of the effects of structural and physical properties on biological activity. Eur. J. Biochem. 268, 5589-5600, 2001). Alternatively, or in addition to, defensins can be incorporated into corneal matrix.

Methods of Incorporation of bioactive agents into cornea matrix:

The following methods may be used:

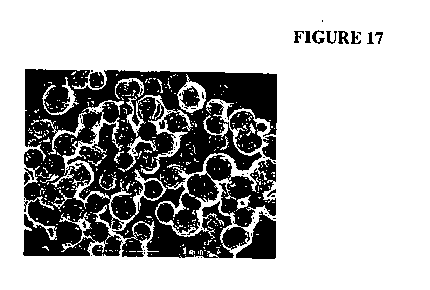

1. Absorption and release
Add IPN hydrogel material into bioactive agent-saturated solution to allow bioactive agents to permeate into cornea matrix. Once the equilibrium is established, the cornea matrix may be used as ocular bandage lens or implant.
2. Chemically grafting into/onto matrix
This procedure is similar to procedure 1 above, but one (or more than one) chemical is used to facilitate the chemical bonding of peptides into/onto IPN hydrogel material. The bioactive peptide-loaded cornea matrix is good for both implant and for bandage lens for drug delivery.
3. Incorporate bioactive agents into nano- or microspheres and incorporate the nano or microspheres into matrix.
This procedure generates an IPN hydrogel matrix with extended release of bioactive agents. The resulting matrix is good for both implant and for bandage lens for drug delivery. Fig. 17 is a scanning electron microscopy (SEM) image of alginate microspheres which are fabricated for encapsulating bioactive agents. After loading bioactive agents, these microspheres are incorporated into matrix for extended drug release.

Procedure for making alginate microspheres Alginate spheres are prepared according to the literature (C.C. Ribeiro, C.C. Barrias, M.A. Barbosa Calcium phosphate-alginate microspheres as enzyme delivery matrices, Biomaterials 25 4363-4373, 2004).

**EXAMPLE XIX: *In vitro* biodegradation of collaaen-MPC IPN hydrogels**

Procedures:

50-80 mg of hydrated hydrogels were placed in vials containing 5 ml 0.1 M PBS (pH 7.4), followed by addition of 60 ul of 1 mg/mL of collagenase (Clostridium histolyticum, EC 3.4.24.3, SigmaChemical Co.). Then the vials were incubated in an oven at 37 ° C, and at different time intervals, the gels were taken out for weighing with surface water wiping off. Time course of residual mass of hydrogels was tracked based on the initial swollen weight. Three specimens were tested for each hydrogel sample. The percent residual mass of hydrogels was calculated in terms of the following equation:

$$\text{Residual mass } \% = W_t / W_o$$

where $W_o$ is the initial weight of the hydrogel and $W_t$ is the weight of the hydrogel at each time point.

Results and discussion

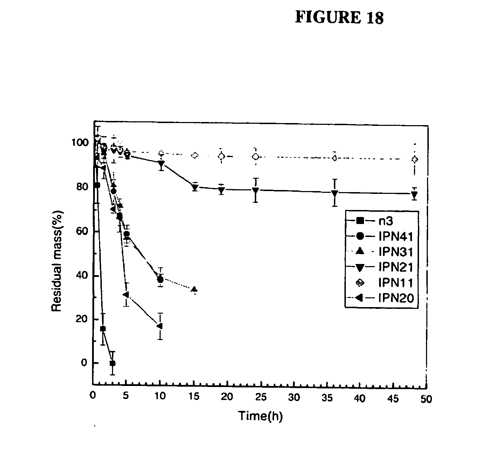

Time course of in vitro biodegradation of collagen and IPN hydrogels
As shown in Figure 18, porcine collagen hydrogel cross-linked with EDC/NHS degraded very fast. After 3h, it was degraded completely. Incorporating MPC and PEG slowed down the degradation rate with IPN-4-1 and IPN-3-1 complete degradation after 10 h and 15 h, respectively. Upon further increasing MPC contents in hydrogels, that is for IPN-2-1 and IPN-I-1, the degradation was significantly suppressed. Within 48 h, the residual mass of IPN-2-1 remained around 79%, whereas IPN-1-1 lost only 6% mass. After 48h, their residual mass was further tracked for 7 day. It was found that both IPN-2-1 and IPN-1-1 hydrogels remained constant in their residual mass. Thus IPN networks is effective in enhancing the biostabilty of collagen hydrogel.

**EXAMPLE XX: Properties of type III collagen-MPC IPN hydrogel and In vitro biodegradation**

Preparation of type III recombinant human collagen (rhc)-MPC IPN hydrogel

Firstly, 0.3 ml of 13.7 wt% rhc III solution and 0.1 ml of MES(0.625 M) were mixed in two syringes connected with a plastic Tee in ice-water bath. Secondly, 250 ul of MPC solution (in MES, MPC/collagen=2/1, w/w) was injected into the above mixture via a 500 ml microsyringe. Thirdly, 9.3 ml of PEG-diacrylate in weight ratio to MPC of 1:2 was injected via a 100 ml microsyringe. Then the solution was thoroughly mixed. Fourthly, 25 ml of 2% APS and TEMED solution(in MES) was injected via a 100 ml microsyringe, followed by injection of 19 ml of EDC/NHS solution(in MES) in molar ratio to collagen $NH_2$ of 1:1:1. The homogenous mixture was cast into glass molds and left at room temperature with 100% humidity under N2 for 24 h. Then the molds were transferred into an incubator for post-curing at 37 °C for 24 h. The product was coded as Coll-III-MPC-IPN2-1-1.
Other rhc III/MPC IPNs with the same condition except for EDC/NHS/Coll-$NH_2$=3/3/1 were prepared. The codes Coll-III-MPC-IPN4-1-3, Coll- III-MPC-IPN3-1-3, oll-III-MPC-IPN2-1-3, Coll-III-MPC-IPN1-1-3 denote IPNs from rhc III/MPC = 4/1, 3/1, 2/1 and 1/1(w/w), respectively.

Mechanical and optical properties

Tables 14 and 15 show the mechanical and optical properties of type III collagen-MPC IPN hydrogels.

Table 14. Mechanical Properties

| Samples | Coll-III-MPC-IPN4-1-3 | Coll-III-MPC-IPN3-1-3 | Coll-III-MPC-IPN2-1-3 | Coll-III-MPC-IPN I-1-3 | Coll-III-MPC-IPN2-1-1 |
|---|---|---|---|---|---|
| Tensile strength (KPa) | 469.4±82.7 | 470.3± | 481.4±95.5 | 456.9±66.4 | 805.1±14.4 |
| Average Break Strain(%) | 25.50±3.91 | 31.14±5.23 | 33.77±8.59 | 20.16±7.29 | 34.11±11.49 |
| Average Modulus(MPa) | 4.788±1.431 | 4.639±1.236 | 4.509±0.724 | 5.457±2.554 | 5.771±1.122 |

Table 15. Optical Transmission

| Wavetength(nm) | White | 450 | 500 | 550 | 600 | 650 |
|---|---|---|---|---|---|---|
| Average Transmission (%) | | | | | | |
| Coll-III-MPC -IPN2-1-1 | 98.3±1.2 | 91.4±3.6 | 93.8±2.4 | 97.5±1.5 | 98.6±2.9 | 99.1±1.7 |

*In vitro* Biodegradation:

The in vitro biodegradation procedures for collagen type III-MPC IPN hydrogel is the same as for the porcine collagen-MPC IPN hydrogels. Only Coll-III-MPC-IPN2-1-1 was tested for in vitro biodegradation. The gel was stable in collagenase solution (12 $\mu$g/mL) for at least 20 days.

**EXAMPLE XXI: Algiante grafted macromolecules.**

In this example, we have developed a two-stage plasma-assisted surface modification technique to covalently graft alginate macromolecules to the posterior surface of collagen-based artificial cornea matrices to prevent endothelial cell attachment and proliferation.

Freeze dried porcine type I collagen powder was obtained from Nippon Ham (Japan) and was dissolved readily in cold water (sterile $_{dd}$ $H_2O$) and stirred at 4°C to give 10% (w/w) concentration. 1-ethyl-3-(3-dimethylaminopropyl) carbodiimide (EDC) and N-hydroxysuccinimide (NHS) were purchased from Sigma-Aldrich. The 3% chitosan solution was prepared by dissolving chitosan powder (MW 40,000Da obtained from Fluka) in 0.2 N hydrochloric acid (HCl) and stirring at 4°C

The 10% collagen solution, 3% (w/w) chitosan solution, and the EDC/NHS cross-linker were mixed together in a syringe system at predetermined ratios to make a homogeneous blend as shown in Table 1.

**Table 1** Chemical composition of corneal hydrogels.

| Cross-linker | Initial collagen concentration (wt/v) % | Chitosan:Collagen molar ratio | Cross-linker to Collagen-NH2 molar equivalent ratio EDC: NHS: NH$_2$*. |
|---|---|---|---|
| **EDC/NHS** | **10** | **1** | **3:2:1** |

The results obtained from endothelial cell growth test showed that alginate surface grafting deterred endothelial cells migration and adhesion to the corneal hydrogels' posterior surfaces. Figure 20 shows number of endothelial cells attached to the posterior surfaces of corneal materials on day five post-seeding as a function of substrate material, plasma power, and alginate solution concentration. General inhibition of endothelial cell growth was observed for all alginate-grafted surfaces, when compared to control surfaces. Control unmodified surfaces are denoted by RF power and alginate concentration of zero in all figures and tables.

As depicted in Figure 20, an interval plot that shows the variation of group means by plotting confidence intervals, the surfaces treated at a plasma power of 100 W and alginate concentration of 5% appeared to effectively inhibit endothelial cell growth by 99%, while the ones treated at a plasma power of 40 W and alginate concentration of 5% appeared to deter endothelial cell growth by about 89%.

All publications, patents and patent applications mentioned in this Specification are indicative of the level of skill of those skilled in the art to which this invention pertains and are herein incorporated by reference to the same extent as if each individual publication, patent, or patent applications was specifically and individually indicated to be incorporated by reference.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

**Claims**

1. An implantable, optically clear, ophthalmic device, wherein the device is a corneal onlay, corneal inlay, or full-thickness corneal implant, and comprises a hydrogel material comprising an interpenetrating network of two or more polymer networks, wherein said interpenetrating network comprises:

   a first network formed by cross-linking a first biopolymer that is entangled with
   a second network.

2. The device according to claim 1, wherein the first biopolymer is collagen, denatured gelatin, fibrin-fibrinogen, elastin, glycoprotein, polysaccharide, glycosaminoglycan, proteoglycan, or oxidized polysaccharide or any combination thereof.

3. The device according to claim 2, wherein the collagen is Type I collagen, Type II collagen, Type III collagen, Type IV collagen, Type V collagen, Type VI collagen, denatured collagen or recombinant collagen of any of the preceding types.

4. The device according to claim 2, wherein the polysaccharide is alginate, chitosan, N-carboxymethyl chitosan, O-carboxymethyl chitosan, N,O-carboxymethyl chitosan, hyaluronic acid or chondroitin sulphates.

5. The device according to claim 2, wherein the oxidized polysaccharide is oxidized chondroitin sulphate, oxidized alginate or oxidized hyaluronic acid.

6. The device according to claim 1, wherein the second polymer network is based on a synthetic polymer or a biopolymer.

7. The device according to claim 6, wherein the synthetic polymer includes alkyl acrylamide, water soluble polyethylene glycol diacrylate, acrylic acid and its derivatives, alkyl acylate, methylacrylic acid and its derivatives, alkyl methacrylate, 2-hydroxyethyl methacrylate, 2-methacryloyloxyethyl phosphorylcholine, vinyl pyrrolidone or glycomonomer as monomers.

8. The device according to any one of claims 1 to 7, wherein the device is **characterized by** at least one of acceptable cytotoxicity, no cytotoxicity, an ability to facilitate cell and/or nerve growth, an ability to withstand handling, implantation, suturing or post-installation wear and tear.

9. The device according to anyone of claims 1 to 8 additionally comprising a bioactive agent or a drug.

10. The device according to claim 9, wherein the device is a corneal onlay and wherein the device is effective in facilitating re-epithelialization over the anterior surface of the device.

11. The device according to anyone of claims 1 to 8 for use in drug delivery.

12. A method of producing a device according to anyone of claims 1 to 10, the method comprising, combining a first polymer network with a second polymer network, wherein at least one of the first polymer network and the second polymer network is based on a biopolymer and combining said first and second polymer network at an acidic pH with at least one cross-linking agent.

13. A device according to anyone of claims 1 to 11, which device is suitable for administration to a mammal.

14. The device according to claim 13, wherein said device comprises a bioactive agent or a drug for delivery to said mammal.

**15.** The device according to claim 14, wherein said bioactive agent or drug is dispersed within the hydrogel material.

**16.** The device according to claim 14, wherein the drug or bioactive agent is contained within nano- or microspheres dispersed within the hydrogel material.

**17.** The device according to claim 16, wherein the bioactive agent is a growth factor, retinoid, enzyme, cell adhesion factor, extracellular matrix glycoprotein, hormone, osteogenic factor, cytokine, antibody, antigen, biologically active protein, pharmaceutical compound, peptide, fragments or motifs derived from biologically active protein, anti-bacterial agent or anti-viral agents.

FIGURE 1

FIGURE 2

# FIGURE 3

# FIGURE 4A

Human Corneal Epithelial Cell Growth on IPN- I Hydrogel

# FIGURE 4B

**Growth of Human Epithelial Cells on IPN-II Hydrogel**

FIGURE 5

FIGURE 6

## FIGURE 7

Top: unoperated cornea
Bottom: Cornea with implant

FIGURE 8

(A)

(B)

(C)

FIGURE 9

**FIGURE 10**

FIGURE 11

(a) Control (b) HPN-3

(c) HPN-7 (d) HPN-5

FIGURE 12

FIGURE 13

EP 2 535 041 A1

## FIGURE 14

FIGURE 15

FIGURE 16

**FIGURE 17**

FIGURE 18

FIGURE 19

**FIGURE 20**

Europäisches
Patentamt

European
Patent Office

Office européen
des brevets

# EUROPEAN SEARCH REPORT

**Application Number**

EP 12 00 5906

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/014969 A1 (OTTAWA HEALTH RESEARCH INST [CA]; CANADA NAT RES COUNCIL [CA]; GRIFFIT) 19 February 2004 (2004-02-19) | 1-4,6-17 | INV.<br>A61K9/00<br>A61L27/14 |
| Y | * examples 4,11 * | 1-17 | C08F283/00<br>C08J3/24 |
| X | US 5 112 350 A (CIVERCHIA LINDA [US] ET AL) 12 May 1992 (1992-05-12) | 1-4,6-17 | C08L5/00<br>C08L89/00 |
| Y | * examples 1,2,5 * | 1-17 | |
| X | US 6 030 634 A (WU CHI [HK] ET AL) 29 February 2000 (2000-02-29) | 1-17 | |
| Y | * example 1 * | 1-17 | |
| Y | US 4 678 468 A (HIROYOSHI TOSHIKI [JP]) 7 July 1987 (1987-07-07) * examples 1-3 * | 1-17 | |
| Y | Anna Gutowska ET AL: "Thermosensitive Interpenetratirig Polymer Networks: Synthesis, Characterization, and Macromolecular Release", Macromolecules, 1 June 1994 (1994-06-01), pages 4167-4175, XP55044124, Retrieved from the Internet: URL:http://dfpcorec-p.internal.epo.org/wf/storage/13AFDE47FCF00004185/originalPdf [retrieved on 2012-11-14] * the whole document * | 1-17 | **TECHNICAL FIELDS SEARCHED (IPC)**<br><br>A61K<br>C08L<br>A61L<br>C08F |

-/--

The present search report has been drawn up for all claims

| Place of search | Date of completion of the search | Examiner |
|---|---|---|
| Munich | 14 November 2012 | Friedrich, Christof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                              

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 12 00 5906

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LIU W ET AL: "Collagen-phosphorylcholine interpenetrating network hydrogels as corneal substitutes", BIOMATERIALS, ELSEVIER SCIENCE PUBLISHERS BV., BARKING, GB, vol. 30, no. 8, 1 March 2009 (2009-03-01), pages 1551-1559, XP025876351, ISSN: 0142-9612, DOI: 10.1016/J.BIOMATERIALS.2008.11.022 [retrieved on 2009-01-19] * the whole document * ----- | 1-17 | |

TECHNICAL FIELDS
SEARCHED (IPC)

The present search report has been drawn up for all claims

| Place of search | Date of completion of the search | Examiner |
|---|---|---|
| Munich | 14 November 2012 | Friedrich, Christof |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 ..........................................................................

& : member of the same patent family, corresponding
    document

**ANNEX TO THE EUROPEAN SEARCH REPORT**
**ON EUROPEAN PATENT APPLICATION NO.**                    EP 12 00 5906

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2012

| Patent document cited in search report | | Publication date | Patent family member(s) | | Publication date |
|---|---|---|---|---|---|
| WO 2004014969 | A1 | 19-02-2004 | AT | 356838 T | 15-04-2007 |
| | | | AU | 2003254674 A1 | 25-02-2004 |
| | | | BR | 0313726 A | 14-06-2005 |
| | | | CN | 1688622 A | 26-10-2005 |
| | | | DE | 60312525 T2 | 06-12-2007 |
| | | | EP | 1530600 A1 | 18-05-2005 |
| | | | EP | 1741457 A1 | 10-01-2007 |
| | | | ES | 2283849 T3 | 01-11-2007 |
| | | | HK | 1078886 A1 | 14-08-2009 |
| | | | JP | 2006516038 A | 15-06-2006 |
| | | | JP | 2008093451 A | 24-04-2008 |
| | | | KR | 20050083626 A | 26-08-2005 |
| | | | MX | PA05001570 A | 19-08-2005 |
| | | | US | 2006134050 A1 | 22-06-2006 |
| | | | US | 2006246113 A1 | 02-11-2006 |
| | | | WO | 2004014969 A1 | 19-02-2004 |
| US 5112350 | A | 12-05-1992 | NONE | | |
| US 6030634 | A | 29-02-2000 | NONE | | |
| US 4678468 | A | 07-07-1987 | JP | 1859003 C | 27-07-1994 |
| | | | JP | 5075429 B | 20-10-1993 |
| | | | JP | 61045765 A | 05-03-1986 |
| | | | US | 4678468 A | 07-07-1987 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5716633 A
- US 4388428 A
- US 4452929 A

### Non-patent literature cited in the description

- **ZENG et al.** *J. Biomech.,* 2001, vol. 34, 533-537
- **H. ULUDAG et al.** *J. Appl. Polym. Sci.,* 2000, vol. 75, 583-592
- **PRIEST ; MUNGER.** *Invest. Ophthalmol. Vis. Sci.,* 1998, vol. 39, 352
- Modem approaches to wettabilty: theory and applications. Plenum Press, 1992, 231-248
- **KONNO T ; HASUDA H ; ISHIHARA K ; ITO Y.** Photo-immobilization of a phospholipid polymer for surface modification. *Biomaterials,* 2005, vol. 26, 1381-1388
- **LEWIS AL.** Crosslinkable coatings from phosphorylcholine-based polymers. *Biomaterials,* 2001, vol. 22, 99-111
- **KLENKLER B.J. ; GRIFFITH M. ; BECERRIL C. ; WEST-MAYS J. ; SHEARDOWN H.** EGF-grafted PDMS surfaces in artificial cornea applications. *Biomaterials,* 2005, vol. 26, 7286-7296
- **LI, F. ; CARLSSON, D.J. ; LOHMANN, C.P. ; SU-URONEN, E.J. ; VASCOTTO, S. ; KOBUCH, K. ; SHEARDOWN, H. ; MUNGER, M. ; GRIFFITH, M.** Cellular and nerve regeneration within a biosynthetic extracellular matrix: corneal implantation. *Proc. Natl. Acad. Sci. USA,* 2003, vol. 100, 15346-15351
- **LI, F. ; GRIFFITH, M. ; LI, Z. ; TANODEKAEW, S. ; SHEARDOWN, H. ; HAKIM, M. ; CARLSSON, D.J.** Recruitment of multiple cell lines by collagen-synthetic copolymer matrices in corneal regeneration. *Biomaterials,* 2005, vol. 26, 3039-104
- **GIANGASPERO, A. ; SANDRI, L. ; TOSSI, A.** Amphipathic alpha helical antimicrobial peptides. A systematic study of the effects of structural and physical properties on biological activity. *Eur. J. Biochem.,* 2001, vol. 268, 5589-5600
- **C.C. RIBEIRO ; C.C. BARRIAS ; M.A. BARBOSA.** Calcium phosphate-alginate microspheres as enzyme delivery matrices. *Biomaterials,* 2004, vol. 25, 4363-4373